# EUROPEAN PATENT SPECIFICATION

(11) **EP 1 545 605 B1**
(45) Date of publication and mention of the grant of the patent: **25.11.2009**
(21) Application number: 03750381.0
(22) Date of filing: 02.10.2003
(51) Int. Cl.: A61K 39/39, A61L 15/44

(54) **COMPOSITION FOR VACCINATION**
ZUSAMMENSETZUNG ZUR IMPFUNG
COMPOSITION VACCINALE

(30) Priority: 02.10.2002 DK 200201473; 02.10.2002 US 415102 P
(43) Date of publication of application: 29.06.2005
(73) Proprietor: Nordic Vaccine Technology A/S, 2880 Bagsvaerd (DK); Coloplast A/S, 3050 Humlebaek (DK)
(72) Inventor: KIKRBY, Nikolai, Soren, DK-2200 Copenhagen N (DK); SAMUELSEN, Peter, Borman, DK-2960 Rungsted Kyst (DK)
(74) Representative: Nilausen, Kim
(86) International application number: PCT/DK2003/000654
(87) International publication number: WO 2004/030696

(56) References cited:
- WO-A-00/07621
- WO-A-92/06710
- WO-A-92/21331
- WO-A-98/36772
- WO-A-98/56420
- WO-A-99/11247
- WO-A-02/074325
- WO-A-02/080981
- WO-A1-99/43350

## Description

### FIELD OF THE INVENTION

The present invention relates to novel constructs for transdermal delivery of at least one immunogen and a process for preparing the constructs as well as use of the constructs.

### BACKGROUND OF THE INVENTION

Transdermal drug delivery (TDD) is a well-known and economically important and attractive administration route for many drugs, such as nicotine in smoking cessation therapy, isosorbide nitrate or nitro glycerine in angina pectoris therapy and estradiol in hormone replacement therapy. Transdermal administration typically involves the delivery of a pharmaceutical agent for percutaneous passage of the drug over the stratum corneum (horny layer) through the epidermis and dermis of the skin or through a mucosal membrane into the systemic circulation of the patient. Transdermal delivery is accomplished by exposing the drug to the skin or the mucosa of a patient for sustained release of the drug. In practice, TDD patches including the drug may be adhered to the body surfaces. TDD administration may also be accomplished by administration of the drug to the skin as creams, ointments, lotions or the like.

Transdermal drug delivery involves the use of a vehicle for the drug, which vehicle preferable but not necessarily possesses skin-adhering properties. A wide number of adhesive systems also called pressure sensitive adhesives systems are generally used, such as silicone, polyurethane, acrylate and polyisobutylene adhesives. These adhesives are in general non-absorbing, have high occlusivity and high skin adhesion and may accordingly cause incompliance with the patient. Other adhesive systems with water absorbing capacity, which may have a better tolerance for the patient, are for instance hydrocolloid adhesives, hydrogel adhesives, cross-linked hydrogel adhesives and the like.

US patent No. 4 367 732 describes a hydrocolloid adhesive comprising styrene-olefine-styrene block copolymers as matrix molecules compounded with various resin tackyfiers and plasticizers being dispersed in hydrocolloid for use in a skin barrier suitable for e.g. bandage purposes. US patent No. 6 153 215 describes the use of hydrocolloid adhesive for delivery of drugs to skin or wounds.

GB patent application No. 2 115 431 discloses a hydrophilic elastomeric pressure sensitive adhesive comprising at least one water-insoluble, cross-linked polymer as matrix and additional plasticizers. The adhesive is useful in various articles, such as bandages and ostomy devices. WO 93/00076 discloses a carrier system for drugs comprising spherical particles and optionally one or more bio adhesive polymers.

US Patent No. 5 410 016 discloses hydrogels of polymerised and cross linked macromer comprising hydrophilic oligomers having biodegradable monomeric or oligomeric extensions for use as i.a. controlled release of drugs or adhesion formation. US Patent No. 6 410 645 discloses a gel forming macromere comprising at least four covalently linked polymeric blocks. The macromers can be cross linked to form a gel on a tissue surface and are useful in a variety of medical applications including drug delivery and tissue coating.

The use of transdermal delivery for vaccination is only scarcely described in literature until now. Furthermore, transdermal delivery of drugs is influenced by many different parameters among which is the size of the molecules to be delivered. Transdermal delivery is normally achieved only for quite small molecules of sizes less than 1 nanometer. For instance, Insulin having a molecule size of about 1,5 - 2 nanometers, is reported to be very complicated to get to pass the skin membrane.

If a transdermal vaccination route could be provided, it would represent an important alternative to invasive administration, e.g. by way of intramuscular, intradermal or subcutaneous injections. Such injections present a range of disadvantages. They may cause stress, pain and irritation, particularly in the case of repeated injections, including the risk of infection - or may be poorly tolerated. Besides, untrained or unlicensed persons may not administer injections. Moreover, when applying for registration of a vaccine by the authorities, the required documentation of none adverse effects will obviously be more extensive for vaccines for invasive administration than for transdermal administration.

Vaccination in the present context is the process by which the application of a vaccine to an individual induces an immunological response in said individual under non-pathogenic conditions.

The transdermal vaccination route which has been described until now preferably includes the steps wherein (i) the upper part of the skin, i.e. the stratum corneum, is removed by a mechanical treatment (scraped of) and (ii) the skin is moistened before application of the vaccine or alternatively (iii) the vaccine is absorbed in a gaze pad and applied on the skin with an ordinary plaster, these techniques is further described in e.g. Transcutaneous immunization, Glenn GM, Kenney RT. New Generation Vaccines, 3rd Ed. Vol. (in press) and in Advances in vaccine delivery: transcutaneous immunization, Glenn GM, Scharton-Kersten T, and Alving CR. Expert Opinion in Investigational Drugs. Vol. 8 (6) (1999): 797-805, WO 99/43350 and US 2001/0006645 A1.

WO 02/074325 discloses a patch for transcutaneous immunisation comprising a pressure-sensitive adhesive layer, at least one immunologically-active protein of an immunogenic formulation applied to the pressure-sensitive adhesive layer and/or incorporated in the pressure-sensitive adhesive layer and a stabilizer. The formulation may contain a protein with adjuvant activity.

WO 99/11247 discloses a composition for transdermal administration of an immunogen comprising a suspension of biphasic lipid vesicles and entrapped in the biphasic lipid vesicles, an immunogen.

WO 00/07621 discloses an adjuvant composition comprising a sterol and a saponin wherein the adjuvant is in the form of an ISCOM. The adjuvant composition is suitable for transdermal administration.

It is an object of the present invention to provide an improved construct and a smarter, more simple and safe method for transdermal delivery under occlusion of an immunogen to an individual thereby avoiding the adverse effects connected with an invasive administration, such as injection.

It is a further object of the invention to provide such compositions and methods, wherein the delivery is accomplished by the use of a delivery system, such as those described in WO patent application no. WO 02/080981 or ISCOMs as described in e.g. WO 98/36772, WO 92/06710 and WO 98/56420.

It has surprisingly been found that utilizing an occlusion vehicle and the principles according to the present invention can mediate an enhanced delivery of immunogen and can act as a potentiator of the immunogen to the immune active cells (often referred to as professional antigen presenting cells) subsiding beneath mucosal membranes, stratum corneum or beneath endothelia cell membranes. As the delivery system and/or adjuvant applied may assume a particulate shape with a mean size of about 5 to 50, e.g. most commonly between 10 and 40 nanometers (nm) or even larger, it is an unexpected observation that such relatively large particles are capable of demonstrating this enhancing feature. Based on observations regarding delivery of drugs, hormones and proteins it is generally anticipated that compounds larger than one or a few nanometers are incapable of penetrating the mentioned cellular membranes.

### SUMMARY OF THE INVENTION

The present invention relates in a first aspect to a construct for transdermal delivery of at least one immunogen to an individual comprising
a) said at least one immunogen
b) an occlusion vehicle and
c) an immunogen delivery system wherein
the immunogen delivery system is a complex comprising:
i) at least one first sterol and/or at least one second sterol,
   wherein the at least one second sterol is capable of contacting a genetic determinant by means of an interaction selected from an electrostatic interaction and a hydrophobic interaction, and
   wherein the at least one first sterol and/or the at least one second sterol is capable of forming a complex with at least one first saponin and/or at least one second saponin, and
ii) at least one first saponin and/or at least one second saponin,
   wherein the at least one second saponin is capable of contacting a genetic determinant by means of an interaction selected from an electrostatic interaction and a hydrophobic interaction, and
   wherein the at least one first saponin and/or the at least one second saponin is capable of forming a complex with at least one first sterol and/or at least one second sterol, and optionally
iii) at least one contacting group for contacting a genetic determinant by means of an interaction selected from an electrostatic interaction and a hydrophobic interaction,
   with the proviso that the at least one contacting group is present when no second sterol is present in the complex and further optionally
iv) at least one lipophilic moiety.

In connection with the present invention the term transdermal delivery includes delivery through a skin surface as well as through a mucous membrane tissue.

As used in connection with the present invention the term occlusion or occlusion vehicle means any covering of skin or mucosal surfaces which covering allows a water vapour transmission less than 15000 g /m² x 24 hrs in an laboratory test with inverted Paddington cups (British Pharmacopoeia 1993, addendum 1996, page 1943) with water in contact to the covering at 37 C° and a relative humidity of 15% in the surrounding environment. In praxis considerably lower values will be used, e.g. less than 12.000 g /m² x 24 hrs, preferably less than 10.000 g /m² x 24 hrs, more preferred less than 6.000 g /m² x 24 hrs, or less than 3.000 g /m² x 24 hrs, even more preferred less than 1.000 g /m² x 24 hrs, or less than 800 g /m² x 24 hrs, less than 500 g /m² x 24 hrs, less than 300 g /m² x 24 hrs or less than 200 g /m² x 24 hrs. By full occlusion is meant a value by said test less than 100 g/ m² x 24 hrs.

The occlusion may be achieved by any covering in form of e.g. film or foil material being slightly or non permeable to water. The best performance is achieved when such covering is fixed to the body surface by adhesives.

The preferred adhesives are able to absorb moisture and retain it in the matrix. This gives the materials the ability to increase the moisture content in the skin or the mucosa. The elevated level of moisture in the skin will increase the ability of penetration of a drug. This is described in literature, see e.g.Henning Gjelstrup Kristensen: Almen farmaci, Institut for Farmaci, Danmarks Farmaceutiske Højskole, 3. udgave (2000), page 332-333. Still further the addition of enhancers to the formulations of adhesives may add to the penetration rate of the drug. In the case of mucosal delivery enhancers may not play any role as the penetration resistance in the endothelial layer is small compared to one of the stratum corneum in skin. On the other hand it may be appropriate to add an enhancer even for mucosal delivery.

A wide number of constructs comprising the compositions of the invention may be used. This is illustrated on the drawings on which:
Figure 1 illustrates a simple monolithic system where the vaccine is applied topically or homogeneously in a vehicle in this case being a pressure sensitive adhesive (2) and where said adhesive is laminated to a cover film (1) at one outer side and a release liner (3) at the opposed side.
Figure 2 illustrates a reservoir system very similar to that of Figure 1, but in which the vaccine-containing vehicle (2) is surrounded by an adhesive rim (4). (1) is the cover film and (3) the release liner.
Figure 3 illustrates a laminate control system identical to the principles of Figure 1 except that a thin release-controlling barrier (5) (preferably porous and pressure sensitive adhesive) is applied between release liner (3) and the vehicle (2).
Figure 4 illustrates the hybrid between Figure 2 and Figure 3. In this system the vehicle (2) for the vaccine typically will be liquid (water).
Figure 5 illustrates a system where the vehicle (2) containing the vaccine is separated from an adhesive (6) intended solely for fixation of the product to the human tissue. The vehicle (2) will be applied by gentle pressure at the central indentation in the release liner (3) upon the adhesive part immediately before removal from release liner and subsequently use. (1) is the cover film.

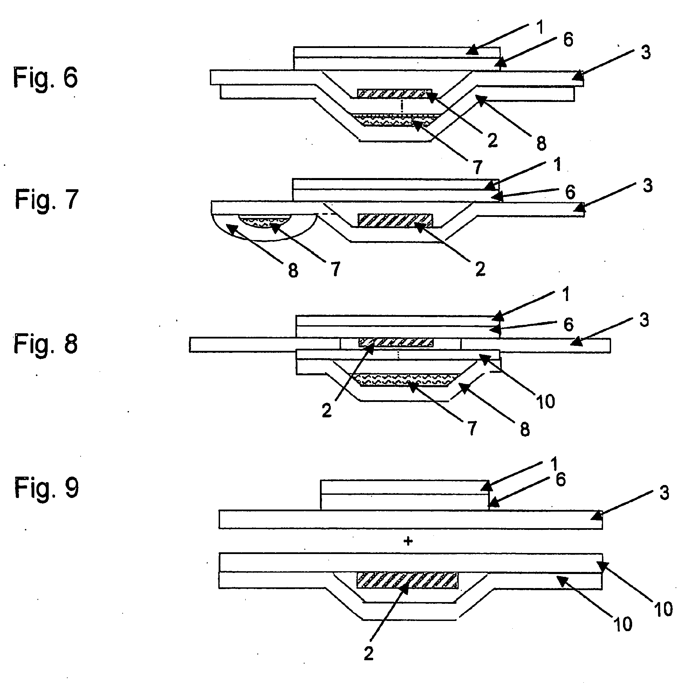

Figure 6 illustrates a water reservoir system. In this concept the vehicle (2) containing the vaccine is a lyophilised pad which is separated from the supporting adhesive (6) by being fixed to the release liner (3). Said liner having a centrally placed weak point (dotted line) for braking. Further a container (8) with water (7) is welded or adhered to the upper surface of the release liner (3). By pressing on the construct the vehicle will adhere to the adhesive (6) in a first step and as a second step the weak point in the liner (dotted line) (3) will break letting the water enter the compartment with the lyophilised vehicle and by doing so the vehicle turns into a gel.
Figure 7 illustrates a principle equivalent to Figure 6 except that that the water containing compartment (7 and 8) is sealed adjacent to the vehicle (2) still with weak release liner (3) for directing the water into the vehicle.
Figure 8 illustrates further an equivalent principle to Figure 6 in which the vehicle (2) is fixed at the support adhesive (6). The membrane (10) is sealed to the release liner (3) and the outer film of the water containing compartment (8) and divide the compartment from the vehicle and has been made weak for breakage by external pressing at the dotted line. The membrane and the release liner (3) may be one and same through indentation of the release liner.
Figure 9 illustrates a two-component packaging for any of above principles in Figure 1 through 5. One component will consist of a film (1) and an adhesive support layer (6) at a release liner (3) and the second component will consist of the vehicle (2) in a pouch (10)

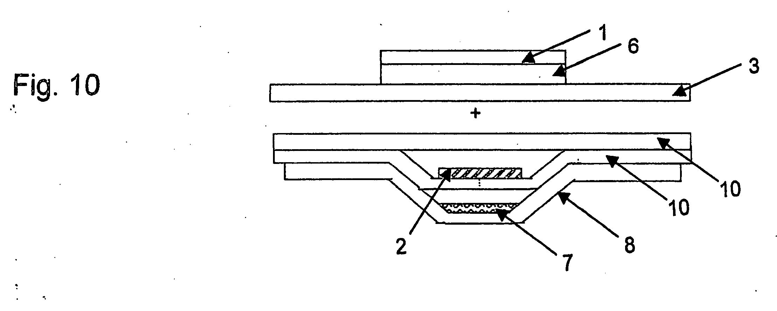

Figure 10 illustrates the two-component principle equivalent to Figure 9 in case of water containing constructs as of Figure 6 through Figure 8. In this case the pouch material (10) is sealed together and has been made weak as illustrated with the dotted line between the vehicle and the water container for directed breakage and water delivery from external pressing.

The present invention also provides in a third aspect a process for the preparation of a composition of the invention, comprising the steps of introducing the immunogen and the immunogen delivery system, which are optionally comprised within a vaccine formulation, into the matrix of the occlusion vehicle or on its surface by dispersion or soaking in a solution of the vehicle or by applying to its surface, and optionally sterilising and/or drying and/or seal packaging the composition.

In other aspects the invention provides the use of a construct of the invention in the manufacture of a transdermal medicament for generating an immunological response in an individual, for treating or preventing a condition of illness in an individual or for vaccination of an individual.

### DETAILED DESCRIPTION OF THE INVENTION

The occlusion vehicle in the construct of the invention is preferable a pressure sensitive adhesive and more preferable an absorbing pressure sensitive adhesive. Such adhesives may especially be found in but are not limited to the group of hydrocolloid adhesives, hydrogel adhesives or cross-linked hydrogels. These adhesive systems are further explained in the following.

### Hydrocolloid Adhesive

The hydrocolloid adhesive is a category of adhesives extensively used for medical devices intended for fixation to the human body. The adhesive is a two phase material consisting of a matrix of adhesive in which are embedded absorbing particles of hydrophilic polymers the so-called hydrocolloids. The principle is quite flexible as the amount and type of particulate hydrocolloids can be tailor made according to the intended application and the base adhesive may be aggressive or easy removable and may further be loaded with suitable enhancers. Also quite many various types of adhesive raw materials may be used in the hydrocolloid adhesives. Such adhesive raw materials may be compounds or blends including polyacrylates, polyisobutylenes, block copolymers having styrene end block, polyurethanes, polyvinyl acetate and the like as commonly known by the person skilled in the art.

### Hydrogel adhesives

In contrast to the hydrocolloid adhesives the hydrogel adhesives are almost entirely moisture sensitive. This means that the adhesive continuously will absorb moisture present in the environment under the change of properties of the adhesive. In particular, Initial absorption will lead to good adhesion to moist surfaces. This may for many purposes be deleterious for its use. However, for one single application and where no carrying of weight is required, the adhesive may be a good alternative, even in moist environments.

The adhesive is classified as a traditional adhesive in which the normally hydrophobic matrix is changed into a hydrophilic. This is achieved by using a scaffold construction of hydrophilic polymers ensuring sufficient cohesion of the adhesive in combination with hydrophilic plasticisers, like polyvalent alcohols and tackifying hydrophilic oligomers. Further some cross-linking physically or chemically may be introduced into the adhesive for improved cohesion of the material. The adhesive is especially suitable for sticking on mucous membranes.

### Hydrogels

The third major group of adhesives for drug delivery is based on hydrogels preferably but not necessarily cross-linked. This group corresponds to the hydrogel adhesive to some extent. The essence is, however, that the plasticiser of the hydrogel is exclusively or mainly water, but it may also include e.g. a polyvalent alcohol like glycerol, polyethylene glycol and polypropylene glycol. Furthermore, the molecular backbone is preferably chemically cross-linked.

The backbone polymers of the hydrogel may essentially be any hydrophilic polymer and preferably such polymers that may be suitable for cross-linking. Preferred are polymers from the category of acrylics like acrylic acid or methacrylates, polyvinyl pyrrolidone, derivatives of polyvinyl pyrrolidone and copolymers of such. However, polyethylene glycols and polyethylene oxide are other often used hydrophilic polymers in cross-linked networks.

The advantage of the hydrogel delivery system may be high solubility of the compound being delivered transdermally, and that the high water content introduced in the skin from the hydrogel leads to increased transdermal penetration. This account when the compound being delivered transdermally is a hydrophilic compound.

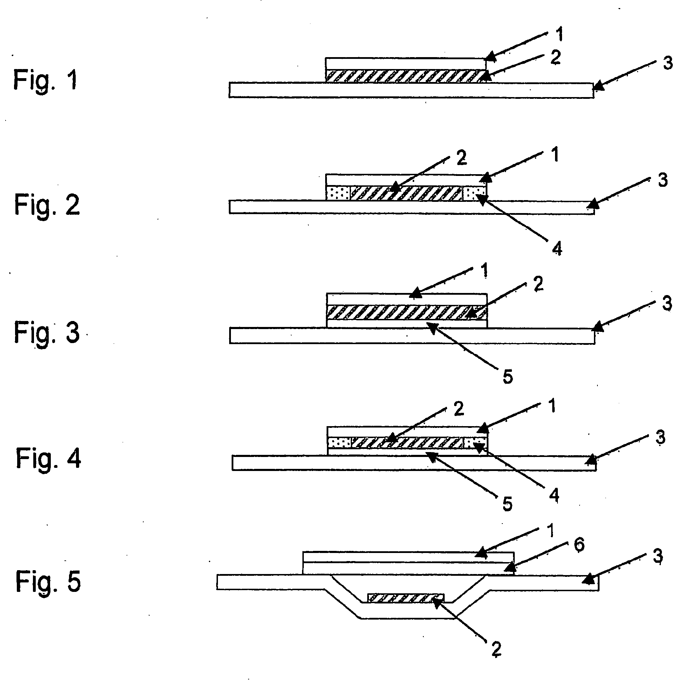

In one embodiment of the invention the immunogen and the immunogen delivery system are distributed In the occlusion vehicle and this distribution is preferably homogenously. This is illustrated in Figure 1, which shows a "monolithic" system.

The immunogen and the immunogen delivery system may in another embodiment also be distributed on the surface of the occlusion vehicle. This will be more efficient and lead to maximal transdermal delivery, as occlusion will lead to decrease in barrier function of the skin and a high concentration of immunogen and immunogen delivery system at the surface of the occlusion vehicle lead to higher instantaneous delivery of immunogen.

As the construct of the invention must be stable over a long period of time it may be preferred to separate the immunogen and the immunogen delivery system from adhesive material, thereby avoiding an exchange of components within the composition. In this embodiment it is also avoided that the adhesive leaves residues, such as plasticisers, in the composition, and that hydrophilic or hydrophobic immunogens/antigens diffuses into the total adhesive.

In this embodiment, which may overcome stability issues, the immunogen and the immunogen delivery system may be embedded in a non-adherent vehicle like for instance a dried or lyophilised hydrophilic polymer substance or in a grease like composition, which potentially may be activated just before use. This activation may happen by the introduction of water solution or other appropriate solvent/diluent to the system. The system will in any case be fixed to the body by a covering, preferably a pressure sensitive adhesive. Accordingly, the construct of the invention in another preferred embodiment comprises a non-adherent occlusion vehicle and a secondary adhesive, being separated from the vehicle, for skin fixation.

Another way to overcome the stability issue may be to position a film barrier between the vehicle for the vaccine and the secondary adhesive.

The construct may also comprise a rate controlling membrane, preferably of a porous and pressure sensitive material, such as a meshed film, open net or the like. The barrier may separate the monolithic system from the patient's skin or mucosa as illustrated in Figure 3.

The occlusion vehicle or the secondary adhesive for skin fixation may be constituted as a covering, e.g. a pad, a dressing or a patch, or be any other common covering known in the art.

The construct of the invention may also comprise an enhancer for transdermal drug delivery. Enhancers in the context of penetration through skin and mucosa are a group of compounds that facilitates the transport of drugs or vaccines over skin or mucous membranes. In respect to the skin the rate-determining layer for permeation is considered to be the horny layer (strateum corneum), which is the very outer layer of the skin. This must be passed whether the target of the drug or vaccine is the skin per se or the full body. This also indicates that use of enhancers for improved penetration is of particular importance when skin is the selected barrier.

Typical enhancers used for trans dermal drug delivery are alcohols, amines, phospholipids, fatty acids, surfactants and polyols. Some particular interesting compounds are low MW-polyethylene glycol, propylene glycol, lauric acid, oleic acid, methyl laurate, ethyl oleate, N-methyl-pyrrolidone, dioctyl adipate and glycerol or low molecular weight derivatives thereof. The requirements to the enhancer will be compatibility with all the present components i.e. the matrix of adhesive or hydrogel and the vaccine components.

The immunogen and the immunogen delivery system are in yet another embodiment separated from each other.

### Application of vaccine and vaccination

Vaccination in the context of the present invention is the process by which the application of a vaccine to an individual induces an immunological response in said individual under non-pathogenic conditions.

### Immunogen and antigen

The construct of the invention may comprise several components, but always at least one immunogen. Preferably, the immunogen is selected in such a way that the induced immunological response is directed against one or more antigens e.g. derived from a pathogenic microorganism. However, the antigens against which the immunological response is directed may be of non-microbial origin; examples of such antigens are synthetic antigens, antigens derived from said individual or antigens derived from any species.

Preferably, the induced immunological response confers protection in said individual against a pathogenic microorganism of which said antigen is part of. Preferably, the induced immunological protection may act upon subsequent exposure of said treated individual to said pathogenic microorganism. However, said vaccinated individual may have been infected by said pathogenic microorganism before the time of application of the vaccine. In this case the protective immunological response may be functional immediately.

By the term induced immunological response is understood a protective response, where the conferred immunological response may be complete protection, leading total elimination of said pathogenic microorganism, however the protection may be partial and only reduce the propagation of said pathogenic microorganism. Furthermore, as the term protection only relates to the reduction or elimination of the state of ill-health induced by said pathogenic microorganism viz. the condition to treat and/or the condition to prevent according to the present invention, the protection may not only be limited to full or partly elimination of said pathogenic. Example of such a mode of action is a protective immunological response directed against a pathogenic component produced by said pathogenic microorganism during the infection of said individual. Examples of such pathogenic components include, but are not limited to bacterial toxins e.g. tetanus toxin.

In connection with the present invention the immunogens are components against which it is possible to raise an immune response in an individual or components, wherein the products of said components can give rise to an immune response. To obtain the immune response it may be necessary that the immunogen is comprised within a vaccine formulation.

As used in connection with the present invention antigens are components, which can be recognized by an immune response, components, wherein fragments of the components can be recognized by an immune response or components, wherein products of said components or fragments of products of said components can be recognized by an immune response.

An immunogen or antigen is in relation to the present invention an immunogen or an antigen preferably derived from a component naturally associated with the condition, which is desirable to treat or prevent according to the present invention. Accordingly, immunogens or antigen which are not normally present within cells or associated with cells of the treated individual and are desirable to target in order to treat or prevent a specific condition, are to be considered as immunogens or antigens with respect of that particular condition.

Preferably, immunogens or antigens are immunogens or antigens, which are not derived from the species, which is to be treated. However, in particular embodiments of the present invention the foreign immunogen or foreign antigens may be derived from the species, which is to be treated. In these embodiments, the immunogen or antigen should not normally be present or associated with said individuals cells.

In one preferred embodiment, the immunogen and/or antigen is not derived from a human being, such as the immunogen or antigen will not be considered as a self-immunogen, when it is administrated to a human being.

In one embodiment of the present invention the immunogen and/or antigen comprises a polypeptide or a peptide, for example the immunogen and/or the antigen may essentially consist of or consist of a polypeptide or a peptide. The immunogen or antigen may also comprise more than one different polypeptide and/or peptide, such as 2, for example 3, such as 4, for example 5, such as 6, for example 7, such as 8, for example 9, such as 10, for example more than 10 different polypeptides.

In some embodiments of the present invention, the immunogen and/or antigen comprises or essentially consists of an organism, preferably a microorganism or part of an organism, preferably a microorganism and accordingly the immunogen or antigen may comprise a very large number of different polypeptides, such as more than 100, for example more than 500, such as more than 1000, for example more than 2500.

It is also contained within the present invention that immunogens or antigens may essentially consist of or consist of one or more polypeptides and/or peptides.

In order to raise an immune response or to enabling an immune response the polypeptides may be processed into fragments and the fragments of the polypeptides may be the compounds, which are actually recognised by the immune response.

Polypeptides as used in connection with the present invention may furthermore comprise posttranslational modifications, such as for example phosphorylation, acetylation, methylation, glycosylation or any other posttranslational modification. In particular, in one embodiment of the present invention the immunogen and/or antigen may comprise a glucosylated polypeptide and/or peptide.

In one preferred embodiment of the present invention the immunogen and/or the antigen comprises a lipopeptide, such as a peptide or a polypeptide chemically linked to a lipid moiety, for example the immunogen and/or the antigen may essentially consist of or consist of a peptide or a polypeptide chemically linked to a lipid moiety.

In another embodiment of the present invention the immunogen or antigen comprises a nucleic acid sequence, for example the immunogen or antigen may essentially consist of or consist of a nucleic acid sequence. The immunogen or antigen may comprise more than one different nucleic acid sequence, such as 2, for example 3, such as 4, for example 5, such as 6, for example 7, such as 8, for example 9, such as 10, for example more than 10 different nucleic acid sequences. In some embodiments the immunogen or antigen may essentially consist of or consist of one or more nucleic acid sequences.

Preferably, the nucleic acid sequences may encode a polypeptide and/or peptide. When the nucleic acid sequence encodes a polypeptide and/or a peptide, preferably, the polypeptide and/or peptide and/or fragments thereof constitute the compound, which is recognised by the immune response.

Accordingly, the following scenario may take place:
i) Nucleic acid sequences are targeted to the target cell
ii) Nucleic acid sequences are intemalised into the target cell
iii) Polypeptides and/or peptides are produced within the target cell
iv) Polypeptides and/or peptides and/or fragments thereof are displayed at the cell surface

Preferably, the polypeptides and/or peptides which are comprised within the immunogen or which are encoded by nucleic acid sequences comprised within the immunogen are foreign to the human body. Preferably, the polypeptides and/or peptides which are comprised within the antigen or which are encoded by nucleic acid sequences comprised within the antigen are foreign to the human body.

In yet another embodiment of the present invention the immunogen and or the antigen comprises a polysaccharide and/or oligosaccharide. Polysaccharides and oligosaccharides comprise at least two monosaccharides, which may be identical or different. The empirical formula of a oligosaccharide is (CH₂O)ₙ and range in size from trioses (n=3) to heptoses (n=7). Polysaccharides within the scope of the present invention may also be branched polysaccharides.

In one preferred embodiment of the present invention the immunogen or antigen is derived from a virus. In another preferred embodiment of the present invention the immunogen or antigen is derived from a bacteria. In yet another preferred embodiment of the present invention the immunogen or antigen is derived from a parasite. In another preferred embodiment of the present invention the immunogen or antigen is derived from a fungus. However, the immunogen or antigen may also comprise a mixture of one or more immunogens and/or antigens selected from the group consisting of viruses, bacteria, fungi and parasites.

The immunogen and/or antigen may for example be an attenuated virus, an attenuated bacteria, an attenuated fungi or an attenuated parasite. Alternatively, the immunogen or antigen may be an inactivated or a killed microorganism selected from the group consisting of viruses, bacteria, fungi and parasites. Mixtures thereof are also contained within the present invention.

Attenuation may for example be accomplished by selecting mutants that have lost pathogenicity after being cultivated for several generations in an unnatural host or after mutagenesis or by manipulation of the microorganism using recombinant DNA techniques. Any other suitable method known to the person skilled in the art may also be used for attenuation.

Inactivation and/or killing of micororganisms may be accomplished by a number of methods, for example heat inactivation, irradiation, chemical inactivation or any other method known to the person skilled in the art.

The immunogen or antigen may furthermore comprise only a part of a microorganism selected from the group consisting of viruses, bacteria and parasites. For example such a part may be a viral capsid, virosome or a ligation of a bacterial or yeast cell membrane Alternatively, the immunogen or antigen may only comprise one or more molecules, which have been derived from viruses, bacteria, fungi and parasites, such as for example polypeptides, peptides or nucleic acid sequences.

Furthermore, the immunogen or antigen may comprise molecules such as for example polypeptides, peptides or nucleic acid sequences, which comprise only fragments of viral, bacterial, fungi and parasite derived polypeptides, peptides or nucleic acid sequences. Such molecules may comprise more than one fragment. Such molecules may also be chimeric, such as they in addition comprise fragments which are not derived from a viruses, bacteria, fungi and/or parasites or fragments which are derived from another virus, bacteria, fungi and/or parasite.

Additionally, the polypeptides, peptides or nucleic acid sequences derived from viruses, bacteria, fungi and parasites may have been manipulated, for example using recombinant DNA techniques, such as the polypeptides, peptides or nucleic acid sequences are not the naturally occurring molecules, but rather derivatives or mutants thereof. Mutants include mutants, which comprise substitutions, deletions and/or additions of amino acids or nucleic acids depending on the character of the molecule.

Viruses may according to the present invention for example be selected from the group consisting of: Adeno-associated virus, Adenovirus, Avian infectious bronchitis virus, Baculovirus, Chicken pox, Monkey Pox, Avi Pox, Corona virus, Cytomegalovirus, Distemper, Enterovirus, Epstein Barr virus, Feline leukemia virus, Flavivirus, Foot and mouth disease virus, Hepatitis A, Hepatitis B, Hepatitis C, Hepatitis E, Herpes species, Herpes simplex, Influenza virus, HIV-1, HIV-2, HTLV 1, Influenza A and B, Kunjin virus, Lassa fever virus, LCMV (lymphocytic choriomeningitis virus), lentivirus, Measles, Mengo virus, Morbillivirus, Myxovirus, Papilloma virus, Parovirus, Parainfluenza virus, Paramyxovirus, Parvovirus, Poko virus, Polio virus, Polyoma tumour virus, pseudorabies, Rabies virus, Reovirus, Respiratory syncytial virus, retrovirus, rhinovirus, Rinderpest, Rotavirus, Semliki forest virus, Sendai virus, Simian Virus 40, Sindbis virus, SV5, Tick borne encephalitis virus, Togavirus (rubella, yellow fever, dengue fever), Vaccinia virus, Venezuelan equine encephalomyelitis and Vesicular stomatis virus.

Preferably, the virus is selected from the group consisting of influenza viruses, herpes viruses, morbili viruses, myxo- and paramyxoviruses, flaviviruses, papillomaviruses and hepatitis viruses.

Bacterias may according to the present invention for example be selected from the group consisting of Achromobacter xylosoxidans, Acinetobacter calcoaceticus, preferably A. anitratus, A. haemolyticus, A. alcaligenes, and A. Iwoffii, Actinomyces israelii, Aeromonas hydrophilia, Alcaligenes species, preferably A. faecalis, A. odorans and A. denitrificans, Arizona hinshawii, Bacillus anthracis, Bacillus cereus, Bacteroides fragilis, Bacteroides melaninogenicus, Bordetella pertussis, Borrelia burgdorferi, Borrelia recurrentis, Brucella species, preferably B. abortus, B. suis, B. melitensis and B. canis, Calymmatobacterium granulomatis, Campylobacter fetus ssp. intestinalis, Campylobacter fetus ssp. jejuni, Chlamydia species, preferably C. psittaci and C. trachomatis, Chromobacterium violaceum, Citrobacter species, preferably C. freundii and C. diversus, Clostridium botulinum, Clostridium perfringens, Clostridium difficile, Clostridium tetani, Corynebacterium diphtheriae, Corynebacterium, preferably C. ulcerans, C. haemolyticum and C. pseudotuberculosis, Coxiella bumetii, Edwardsiella tarda, Eikenella corrodens, Enterobacter, preferably E. cloacae, E. aerogenes, E. hafniae (also named Hafnia alvei) and E. agglomerans, Erysipelothrix rhusiopathiae, Escherichia coli, Flavobacterium meningosepticum, Francisella tularensis, Fusobacterium nucleatum, Gardnerella vaginalis, Haemophilus ducreyi, Haemophilus influenzae, Helicobacter species, Klebsiella species, preferably K. pneumoniae, K. ozaenae og K. rhinoscleromatis, Legionella species, Leptospira interrogans, Listeria monocytogenes, Moraxella species, preferably M. lacunata and M. osloensis, Mycobacterioum bovis, Mycobacterium leprae, Mycobacterium tuberculosis, Mycoplasma species, preferably M. pneumoniae, Neisseria gonorrhoeae, Neisseria meningitidis, Nocardia species, preferably N. asteroides and N. brasiliensis, Pasterurella haemolytica, Pasteurella multocida, Peptococcus magnus, Plesiomonas shigelloides, Pneumococci, Proteus species, preferably P. mirabilis, P. vulgaris, P. rettgeri and P. morganii (also named Providencia rettgeri and Morganella morganii respectively), Providencia species, preferably P. alcalifaciens, P. stuartii and P. rettgeri (also named Proteus rettgeri), Pseudomonas aeruginosa, Pseudomonas mallei, Pseudomonas pseudomallei, Rickettsia, Rochalimaia henselae, Salmonella species, preferably S. enteridis, S. typhi and S. derby, and most preferably Salmonella species of the type Salmonella DT104, Serratia species, preferably S. marcescens, Shigella dysenteriae, S. flexneri, S. boydii and S. sonnei, Spirillum minor, Staphylococcus aureus, Staphylococcus epidermidis, Staphylococcus saprophyticus, Streptobacillus moniliformis, Streptococcus, preferably S. faecalis, S. faecium and S. durans, Streptococcus agalactiae, Streptococcus pneumoniae, Streptococcus pyogenes, Treponema carateum, Treponeam pallidum, Treponema pertenue, preferably T. pallidum, Ureaplasma urealyticum, Vibrio cholerae, Vibrio parahaemolyticus, Yersinia enterocolitica, and Yersinia pestis.

Parasites may according to the present invention for example be selected from the group consisting of Malaria (Plasmodium. falciparum, P. vivax, P. malariae), Schistosomes, Trypanosomes, Leishmania, Filarial nematodes, Trichomoniasis, Sarcosporidiasis, Taenia (T. saginata, T. solium), Leishmania, Toxoplasma gondii, Trichinelosis (Trichinella spiralis) or Coccidiosis (Eimeria species).

The immunogen and/or antigen could furthermore be derived a fungus selected from the group consisting of Cryptococcus neoformans, Candida albicans, Apergillus fumigatus and Coccidioidomycosis.

It is however possible, that the immunogen and/or the antigen is derived from any animal, including for example vertebrates. For example the immunogen and/or antigen may comprise components derived from or may essentially consist of the group consisting of ovalbumin, keyhole limpet hemocyanin and sperm-whale myoglobulin. Other examples include, but are not limited to, immunogens and or antigens derived from bee and snake venoms, and biological active components produced by bloodsucking animals, especially insects, for e.g. inhibition of blood coagulation

The immunogen and/or the antigen may in one embodiment comprise a hapten linked to a carrier molecule, for example the immunogen and/or the antigen may essentially consist of or consist of a hapten linked to a carrier molecule. Alternatively, the product of the immunogen and/or the antigen may comprise or essentially consist of or consist of a hapten linked to a carrier molecule. Haptens are small chemically defined compounds that become immunogenic upon conjugation to a carrier molecule, which however are only weakly immunogenic alone. Examples of haptens are, but not limited to, bacterial cell-wall components linked to protein carriers e.g. bacterial toxins or detoxified bacterial toxins.

The immunogen and/or the antigen may in another embodiment be a multivalent immunogen and/or antigen. Multivalent antigens and/or immunogens may for example be selected from the group consisting of T-independent type 2 antigens, synthetic polymers and multimeric peptide antigens. However the immunogen and/or the antigen may also be a any other multivalent antigen and/or immunogen known to the person skilled in the art.

Preferably, the immunogen and/or the antigen comprises a peptide capable of being presented by MHC molecules, for example the foreign immunogen and/or the foreign antigen may essentially consist of or consist of a peptide capable of being presented by MHC molecules. Alternatively, the products of the immunogen and/or the antigen comprises a peptide capable of being presented by MHC molecules, for example the products of the immunogen and/or the antigen may essentially consist of or consist of a peptide capable of being presented by MHC molecules.

The peptide capable of being presented by MHC molecules may be any such peptide known to the person skilled in the art. Preferred examples of such peptides are listed in the following database: "MHCPEP - A database of MHC binding peptides (v. 1.3)", compiled by Brusic (V. Brusic, G. Rudy, A.P. Kyne and L.C. Harrison; MHCPEP, a database of MHC-binding peptides: update 1997; Nucleic Acids Research, 1998, Vol. 26, No. 1, pp. 368-371).

In addition, the antigen and/or the immunogen may comprise a mixture of two or more of the above-mentioned immunogens and antigens.

Preferably, the antigen may be recognised by an immune response raised against the immunogen. Accordingly, the antigen and the immunogen should preferably resemble one another.

More preferably, they resemble one another so that they comprise compounds, which alone or together with other molecules are capable of associating with the same interacting molecule. Alternatively, they comprise components, wherein fragments of the components alone or together with one or more other molecules are capable of associating with the same interacting molecule. Interacting molecules within this context are preferably components of the immune system. For example interacting molecules may be selected from the group consisting of antibodies and T-cell receptors.

In one embodiment of the present invention the antigen and the immunogen are the same, meaning that the immunogen and the antigen are identical. In another embodiment of the present invention the antigen is a fragment of the immunogen. In yet another embodiment of the present invention the immunogen is a fragment of the antigen. In a yet further embodiment of the present invention the antigen mimics the immunogen.

### Immunological response

The immune response against the immunogen and/or antigen may be present in the individual prior to application to the treated individual of the composition or vaccine formulation according to the present invention. For example such an immune response may have been generated following an infection of said individual. Accordingly, the method according to the present invention includes methods to enhance an existing immunological response and/or biasing an existing immunological response with respect to current or future mode of action (e.g. cellular vs. humoral responses), composition (e.g. antibody classes, antibody subclasses etc.), specificity (e.g. recognition of new or different sub-parts of the immunogen and or antigen) and/or affinity.

In many cases however, the individual is not immune against the immunogen and/or antigen prior to application to the treated individual of the composition or vaccine formulation according to the present invention. Accordingly, the method according to the present invention includes methods of raising an immune response to the immunogen and/or antigen in an individual.

Preferably, the method comprises the steps of:
i) Immunizing an individual with the immunogen; and
ii) Raising an immune response against the immunogen in said individual

In preferred embodiments the immunogen is administrated to the individual, internalized into cells of the individual, processed in said cells and displayed on the surface of said cells.

Immunogenic determinants denote any substance capable of raising an immune response, including a specific antibody response. Any antigenic determinant is thus also an immunogenic determinant, but not all immunogenic determinants are antigenic determinants within the meaning of these terms as used herein. The immunogen may be comprised in a vaccine formulation as described herein.

The immunological response, often termed inflammatory response, according to the present invention may be mediated by any components of the immune system of the treated individual.

The immune system may exhibit both specific and non-specific immunity (Klein, J., et al., Immunology (2nd), Blackwell Science Inc., Boston (1997)). Generally, B and T lymphocytes, which display specific receptors on their cell surface for a given antigen, produce specific immunity. The immune system may respond to different antigens in two ways: 1) humoral-mediated immunity, which includes B cell stimulation and production of antibodies or immunoglobulins [other cells are also involved in the generation of an antibody response, e.g. antigen-presenting cells (APCs; including macrophages), and helper T cells (Th1 and Th2)], and 2) cell-mediated immunity (CMI), which generally involves T cells including cytotoxic T lymphocytes (CTLs), although other cells are also involved in the generation of a CTL response (e.g., Th1 and/or Th2 cells and APCs).

Non-specific immunity encompasses various cells and mechanisms such as phagocytosis (the engulfing of foreign particles or antigens) by macrophages or granulocytes, and natural killer (NK) cell activity, among others.

Non-specific immunity relies on mechanisms less evolutionarily advanced (e.g., phagocytosis, which is an important host defense mechanism) and does not display the acquired nature of specificity and memory, hallmarks of a specific immune response. Nonspecific immunity is more innate to vertebrate systems. In addition, cells involved in nonspecific immunity interact in important ways with B and T cells to produce an immune response.

The key differences between specific and nonspecific immunity are based upon B and T cell specificity. These cells predominantly acquire their responsiveness after activation with a specific antigen and have mechanisms to display memory in the event of future exposure to that specific antigen. As a result, vaccination (involving specificity and memory) is an effective protocol to protect against harmful pathogens.

The cell-mediated may be a cytolytic process or comprise a cytolytic process. Preferably, however the cytotoxic and/or inflammatory response according to the present invention is mediated by cytotoxic T-cells. Such cytotoxic T-cells preferably express T-cell receptors that can associate with the antigen or fragments of the antigen or products of the antigen or fragments of products of the antigen according to the present invention.

However, the cytotoxic and/or inflammatory response may also be mediated by natural killer cells. In addition the cytotoxic and/or inflammatory response may for example be mediated by neutrophils or the cytotoxic and/or inflammatory response may be mediated eosinophils.

Furthermore, the cytotoxic and/or inflammatory response may be mediated by antibody-dependent cell-mediated cytotoxicity (ADCC) mechanisms. Such mechanisms preferably involve antibodies that can associate with the antigen or fragments of the antigen or products of the antigen or fragments of products of the antigen according to the present invention.

In one embodiment of the present invention, the cytotoxic and/or inflammatory response may be mediated by the innate immune system. For example the cytotoxic and/or inflammatory response may be mediated by the complement cascade. For example, the cytotoxic and/or inflammatory response may be mediated by the process of opsonisation by antibodies. For example, the cytotoxic and/or inflammatory response may be mediated by the process of opsonisation by one ore more components of the complement system.

In particular, the cytotoxic and/or inflammatory response may be mediated by of opsonisation of antibodies on the cellular surface of target cells by the classical complement pathway or the cytotoxic and/or inflammatory response may be initiated directly by the activation of complement factors, the alternative complement pathway.

Following the activation of one or both complement pathways the terminal membrane-attached complex may be formed, the target cell may be lysed and eliminated by said complex. In one preferred embodiment of the present invention the cytolytic process may result from the formation of a membrane-attack complex.

Antibodies that may activate the complement pathways according to the present invention, may for example be antibodies selected from the group consisting of IgG1, IgG2, IgG3 and IgM antibody isotypes.

Antibody-dependent cell-mediated cytotoxicity (ADCC) mechanisms may comprise other anti-cellular effectors than the complement pathways. For example antibodies bound to targeted cell may direct cytolytic activities of eosinophiles against the targeted cells. In such an embodiment the antibodies preferably are IgE.

In addition, the cytotoxic and/or inflammatory response may be mediated by a combination of two or more mechanisms mentioned herein above. For example it may be mediated by two or more selected from the group consisting of cytotoxic T-cells, natural killer cells, neutrophils, eosinophils, antibody-dependent cell-mediated cytotoxicity and cytolytic mechanisms.

### Immunogen delivery system

The immunogen delivery system may in principle be any immunogen delivery targeting system capable of being targeted to target cells of an individual, wherein the target cells are desirable to target and/or eliminate. A number of different immunogen delivery targeting systems are known to the person skilled in the art and a suitable immunogen delivery targeting system may be selected according to the specific need. According to the invention, the immunogen delivery system is a PosIntro.

In a preferred embodiment the immunogen and the immunogen delivery system is comprised within a vaccine formulation. Any vaccine formulation known to the person skilled in the art may be used in connection with the present invention.

In one embodiment of the present invention the immunogen delivery system is cationic.

PosIntros may for example be any of the compounds described in the pending WO patent application no. WO 02/080981.

PosIntros within the scope of the present invention are complexes comprising:
i) at least one first sterol and/or at least one second sterol,
   wherein the at least one second sterol is capable of contacting a genetic determinant, preferably a nucleic acid by means of an Interaction selected from an electrostatic interaction and a hydrophobic interaction, and
   wherein the at least one first sterol and/or the at least one second sterol is capable of forming a complex with at least one first saponin and/or at least one second saponin, and
ii) at least one first saponin and/or at least one second saponin,
   wherein the at least one second saponin is capable of contacting a genetic determinant by means of an interaction selected from an electrostatic Interaction and a hydrophobic interaction, and
   wherein the at least one first saponin and/or the at least one second saponin is capable of forming a complex with at least one first sterol and/or at least one second sterol, and optionally
iii) at least one contacting group for contacting a genetic determinant by means of an interaction selected from an electrostatic interaction and a hydrophobic interaction,
   with the proviso that the at least one contacting group is present when no second sterol is present in the complex and further optionally

i) at least one lipophilic moiety.
   PosIntros according to the present invention may in one preferred embodiment adopt a micro-particle structure in the form of a cage-like matrix similar to that known as an immune stimulating complex (iscom). Beside iscom structures, the interaction between sterols and saponins have been reported to result in a variety of different structural entities, including entities such as e.g. lattices, honeycombes, rods, and amorphic particles, all of which structural entities are covered by the present invention.

In one embodiment of the invention the immunogen delivery system comprises a biodegradable microsphere. In another embodiment the immunogen delivery system comprises an encapsulation system. In one preferred embodiment the immunogen delivery system comprises a cochleate. In yet another embodiment the immunogen delivery system comprises a nanoparticle. In a still further embodiment the immunogen delivery system comprises a hydrogel. In an even still further embodiment the immunogen delivery system comprises a microcrystal.

The construct and vaccine formulation may comprise more than one immunogen or antigen, such as 2, for example 3, such as 4, for example 5, such as more than 5 different antigens. The immunogens and antigens may be selected from the group of immunogens and antigens described herein above.

The construct according to the invention may contain conventionally nontoxic pharmaceutically acceptable carriers and excipients. The pharmaceutically acceptable excipients may include adjuvants, penetration adjuvants, emulsifying agents, antioxidants, buffering agents, preservatives, humectants, chelating agents, gel forming agents and the like and are all selected in accordance with conventional pharmaceutical practice in a manner understood by the persons skilled in the art of formulating pharmaceuticals.

Preferably, the construct and vaccine formulation according to the present invention furthermore comprises an adjuvant. The vaccine formulation according to the present invention may furthermore comprise a carrier. The carrier or adjuvant could be any carrier or adjuvant known in the art including functional equivalents thereof. Functionally equivalent carriers are capable of presenting the same antigen in essentially the same steric conformation when used under similar conditions. Functionally equivalent adjuvants are capable of providing similar increases in the efficacy of the composition when used under similar conditions.

Preferably, said formulations comprise potent, nontoxic adjuvants that will enhance and/or modulate the immunogenicity of immunogenic determinants including antigenic determinants including haptenic determinants represent one group of preferred adjuvants. In addition, such adjuvants preferably also elicit an earlier, more potent, or more prolonged immune response. Such an adjuvant would also be useful in cases where an immunogen supply is limited or is costly to produce.

Adjuvants pertaining to the present invention may be grouped according to their origin, be it mineral, bacterial, plant, synthetic, or host product. The first group under this classification is the mineral adjuvants, such as aluminium compounds. Antigens precipitated with aluminium salts or antigens mixed with or adsorbed to performed aluminium compounds have been used extensively to augment immune responses in animals and humans. Aluminium particles have been demonstrated in regional lymph nodes of rabbits seven days following immunisation, and it may be that another significant function is to direct antigen to T cell containing areas in the nodes themselves. Adjuvant potency has been shown to correlate with intimation of the draining lymph nodes. While many studies have confirmed that antigens administered with aluminium salts lead to increased humoral immunity, cell mediated immunity appears to be only slightly increased, as measured by delayed-type hypersensitivity. Aluminium hydroxide has also been described as activating the complement pathway. This mechanism may play a role in the local inflammatory response as well as immunoglobulin production and B cell memory. Furthermore, aluminium hydroxide can protect the antigen from rapid catabolism. Primarily because of their excellent record of safety, aluminum compounds are presently the only adjuvants used in humans.

Another large group of adjuvants is those of bacterial origin. Adjuvants with bacterial origins can be purified and synthesized (e.g. muramyl dipeptides, lipid A) and host mediators have been cloned (Interleukin 1 and 2). The last decade has brought significant progress in the chemical purification of several adjuvants of active components of bacterial origin: Bordetella pertussis, Mycobacterium tuberculosis, lipopolysaccharide, Freund's Complete Adjuvant (FCA) and Freund's Incomplete Adjuvant (Difco Laboratories, Detroit, Mich.) and Merck Adjuvant 65 (Merck and Company, Inc., Rahway, N.J.). Additionally suitable adjuvants in accordance with the present invention are e.g. Titermax Classical adjuvant (SIGMA-ALDRICH), ISCOMS, Quil A, ALUN, see US 58767 and 5,554,372, Lipid A derivatives, choleratoxin derivatives, HSP derivatives, LPS derivatives, synthetic peptide matrixes, GMDP, and other as well as combined with immunostimulants (US 5,876,735).

B. pertussis is of interest as an adjuvant in the context of the present invention due to its ability to modulate cell-mediated immunity through action on T-lymphocyte populations. For lipopolysaccharide and Freund's Complete Adjuvant, adjuvant active moieties have been identified and synthesized which permit study of structure-function relationships. These are also considered for inclusion in immunogenic compositions according to the present invention.

Lipopolysaccharide and its various derivatives, including lipid A, have been found to be powerful adjuvants in combination with liposomes or other lipid emulsions. It is not yet certain whether derivatives with sufficiently low toxicity for general use in humans can be produced. Freund's Complete Adjuvant is the standard in most experimental studies.

Mineral oil may be added to vaccine formulation in order to protect the antigen from rapid catabolism.

Many other types of materials can be used as adjuvants in immunogenic compositions according to the present invention. They include plant products such as saponin, animal products such as chitin and numerous synthetic chemicals.

Adjuvants according to the present invention can also been categorized by their proposed mechanisms of action. This type of classification is necessarily somewhat arbitrary because most adjuvants appear to function by more than one mechanism. Adjuvants may act through antigen localization and delivery, or by direct effects on cells making up the immune system, such as macrophages and lymphocytes. Another mechanism by which adjuvants according to the invention enhance the immune response is by creation of an antigen depot. This appears to contribute to the adjuvant activity of aluminum compounds, oil emulsions, liposomes, and synthetic polymers. The adjuvant activity of lipopolysaccharides and muramyl dipeptides appears to be mainly mediated through activation of the macrophage, whereas B. pertussis affects both macrophages and lymphocytes. Further examples of adjuvants that may be useful when incorporated into immunogenic compositions according to the present invention are described in US 5,554,372.

In one preferred embodiment, adjuvants according to the present invention are selected from the group consisting of aluminium compounds, Freunds incomplete adjuvant, Titermax classical adjuvant and oil emulsions.

There is also provided an embodiment of the present invention wherein the construct or vaccine formulation further comprises a carrier. The carrier may be present independently of an adjuvant. The purpose of conjugation and/or co-immunisatlon of an antigen and a carrier can be e.g. to increase the molecular weight of the antigen in order to increase the activity or immunogenicity of the antigen, to confer stability to the antigen, to increase the biological activity of the determinant, or to increase its serum half-life. The carrier protein may be any conventional carrier including any protein suitable for presenting antigens. Conventional carrier proteins include, but are not limited to, keyhole limpet hemocyanin, serum proteins such as transferrin, bovine serum albumin, or human serum albumin, an ovalbumin, immunoglobulins, or hormones, such as insulin.

The construct or vaccine formulation according to the present invention may furthermore comprise a biological active component. A biological active component may be any component, which directly or indirectly can influence the immune response of an individual, Preferably, the biological active component is selected from the group consisting of cytokines and chemokines.

Cytokines may for example be selected from the group consisting of IL-2, IL-4, IL-10, IL-12, IL-15, IL-18, IL-21, IFN-γ, IFN-α, GM-CSF, C-CSF.
The compositions of the invention may be manufactured in various ways.

For monolithic use of the adhesive the the immunogen and the immunogen delivery system optionally comprised within a vaccine formulation may be introduced into the matrix of the adhesive to a homogeneous distribution by spraying this dispersion onto the particular hydrocolloids before these are mixed within the adhesive matrix. During the spraying the hydrocolloid particles shall be mixed and rotated to secure an even distribution of the immunogen and its delivery system. In a second step the hydrocolloid is mixed at relatively low temperatures into the thermoplastic molten adhesive and in a third step the final blend is coated in the preferred thickness by a conventional coating technique.

In respect to hydrocolloid adhesives the release rate is very slow from such homogeneously distributed immunogen or vaccine components. For cross-linked hydrogels containing homogeneously distributed immunogen/immunogen delivery system or vaccine components the release rate appears to be faster. When the immunogen delivery system is PosIntros, i.e. nanoparticles, it is preferred to apply these to the surface of the adhesives. This is preferably done from an aqueous dispersion and results in an isotropic material with inhomogeneous distribution of the PosIntros. The surface with the high content of PosIntros is intended to be the releasing side of the material.

In other embodiments the immunogen delivery system and the immunogen are used in dry state. This may be obtained by simple drying or lyophilisation of these components. Preferably however a process assistant agent like a hydrophilic polymer is used for creation of better bulk properties. Such agent may be polysaccharides and cellulose material or synthetic polymers like polyvinyl pyrrolidone and polyvinyl alcohol but are not limited to those. Drying solutions may result in brittle pads or porous structures depending on type of process. Better properties may be achieved by adding plasticiser and/or appropriate enhancers for trans dermal drug delivery. The immunogen and the delivery system may also be soaked into porous cross-linked material and in a second step be dried.

The finished products will in general be *sterile*. This implies appropriately sealed packaging chosen according to the selected method of sterilization. The preferred methods of sterilization are beta or gamma radiation. However, autoclaving may be used for hydrogels and ethylene oxide sterilization or plasma sterilization for hydrocolloid and hydrogel adhesives. Autoclaving is the only method that is inappropriate for lyophilised or otherwise dry pads based on hydrophilic polymers.

The construct of the invention has in one embodiment more than one compartment and in another embodiment at least two compartments, wherein a first compartment comprises a lyophilised pad comprising the immunogen and the immunogen delivery system and a second compartment comprises aqueous solution.

### Film and release liners

For practical use the construct of the invention will comprise a cover film and/or a release liner. The covering, e.g. the patch, pad or dressing containing the immunogen or vaccine components comprises a barrier to the side not in contact with the tissue. Such barrier may be a film of a polymeric material like polyurethane, polyethylene, polyamide, polyester, polyvinyl alcohol and the like but should not be restricted to such. The barrier may be of metal foil or even thin ceramics. Accordingly, the choice of the barrier film will not be critical to the invention. Typical the requirement to said barrier material would be flexibility and conformability, for which reason thin polymeric films are preferred.

For protection of the opposite side of the patch release liners based on e.g. silicone and the like will be preferred, but other materials may be used. They may be based on sheets of paper or plastic film. In case of cross-linked hydrogels, plastic films are preferred and siliconising will normally not be required.

Other films, which will be suitable according to the invention, for instance for constructs comprising two component or for constructs comprising a water containing compartment may be quite ordinary for the person skilled in the art; and preferably multilaminate barrier films from plastics are used.

The conditions to be treated and the conditions to be prevented by the compositions of the present invention are conditions of illness of an individual where the progress of a disease can be reduced or eliminated by a recognition of the disease-causing agent by said individuals own immune system after specific induction of a immunological response in said individual.

Typically, the condition is, but not limited to, a disease caused by infection of said individual by a pathogenic microorganism.

### EXAMPLES

### Example 1

### Preparation of vaccine (immunogenlimmunogen delivery system) (experimental Tetanus vaccine)

Mega 10 (N-Decanoyl-N-methylglucamide) was purchased from Sigma-Aldrich, St. Louis, MO and used as a 20% stock solution. Cholesterol, DC-cholesterol (Sigma-Aldrich, St. Louis, MO) and phophatidylcholine (Epikuron 200S, Lucas Meyer Gmbh, Germany) was dissolved in 20% Mega 10 at a concentration of 1 % w/v with respect to each component and stored at -20°C. Quil A saponin was obtained form Superfos Biosector, Frederikssund, Denmark. Quil A was dissolved (15mg/ml) in Milli-Q water and filtered to sterility and stored in solution at -20°C until used.

Tetanus-PosIntro™ was prepared by combining Quil A, cholesterol/DC-cholesterol and phophatidylcholine with Tetanus toxoid (List Biological Laboratories, Inc.). PosIntro™ micro-particles were allowed to form for 4 hours with stirring at 35°C followed by dialysis against phosphate buffered saline (pH 7.2) in Slide-A-Lyzer® cassettes (Pierce, Rockford, IL) or dialysis tubing (Visking, London, UK), MW cut-off 10,000. The concentration in the reaction mixture of Quil A was 1.1 mg/ml and the concentration of cholesterol, DC-cholesterol and phophatidylcholine was 0.03% w/v. The final concentration of Mega 10 was 4% w/v unless otherwise stated. The concentration of Tetanus toxoid in the reaction mixture ranged from 0.1 to 0.5mg/ml, depending on the experiments.

### Example 2

### Purification of Tetanus-PosIntro™.

Sucrose gradients were prepared from 25% (w/v) sucrose in phosphate buffered saline (PBS) pH 7.2. Polyallomer™ centrifuge tubes (size 13x51 mm, Beckmann Instruments) were filled with 4.5ml sucrose solution and stored at -20°C. Upon usage the required number of tubes was allowed to thaw slowly at 4°C, typically overnight, whereby the gradient formed. Gradients were allowed to equilibrate at room temperature for at least 1 hour before Tetanus-PosIntro™-preparations were applied in a volume of 0.5ml or less. Centrifugation was performed in a Beckmann Instruments rotor type SW55Ti at 20°C, 50.000rpm for 3 hours. Intact micro-particles were colleted through a diode array UV-detector (Perkin Elmer) and monitored at 210nm.

Purified Tetanus-PosIntro™ micro particles were dialysed first against phosphate buffered saline (pH 7.2) in Slide-A-Lyzer® cassettes (Pierce, Rockford, IL) in order to remove sucrose. The vaccine preparation was further up-concentrated by reverse dialysis against Slide-A-Lyzer Concentrating Solution™ (Pierce, Rockford, IL). The selected liquid vaccine formulation contained Tetanus toxoid embedded into PosIntro™ micro-particles with sizes of 35-50nm as visualized by transmission electron microscopy. The concentration of Tetanus toxoid was 4.2mg/ml whereas the concentration of Quil A was 12mg/ml.

### Example 3

### Amino acid analysis

The Pico-Tag System® from Waters Corporation was used for determination of protein content of PosIntro™ micro-particles. Hydrolysis was performed in a Pico-Tag workstation at 150°C for 1 hour using 6N HCl and phenol, followed by derivatization with phenylisothiocyanale (PITC) according to the Pico-Tag protocol. In order to obtain quantitative measurement the PosIntro™-structure was disrupted by treatment with 1:1 dichloromethane (DCM) and acetonitril (ACN) for 10 min at room temperature after which solvent/diluent was evaporated in vacuum

### Example 4

### Hydrocolloid adhesive as vehicle for vaccine (immunogen and delivery system)

The hydrocolloid vehicle is produced as follows: In a Z-blade mixer is introduced 45 g of a tackyfier resin (Arkon P90, Arakawa) at 140 °C. After 5 minutes mixing 32,5 g of a block copolymer Styrene-Isoprene elastomer (Kraton TR 1107, Shell) and 5 g dioctyl adipate are added. After further 10 minutes further 7 g dioctyl adipate is added and the mixture is blended until homogeneous. Finally 30 g of carboxy methyl cellulose (Aquasorb, Hercules) is added. The mixture is coated by hot melt coating in a thickness of 0,5 mm at a film of polyurethane (30 µm) coated at a silicone paper.

A solution of the vaccine prepared as described in example (vaccine equivalent to 0.100 mg/ cm²) is applied evenly to circular areas of 15 mm at the surface of the adhesive before drying and followed by laminating with a release-liner. Finally patches are die-cut from the adhesive in round patches with the vaccine area in the centre and in diameters of 30 mm.

The patches are packed individually and sterilized by electron beam radiation.

### Example 5

### Hydrocolloid adhesive as vehicle for vaccine

A hydrocolloid adhesive is produced as described in example 4 except that the dioctyl adipate is replaced as follows: the first addition of adipate is replaced with 10 g paraffin oil and the second is replaced to 15 g paraffin oil. The homogeneous mass is hot melt coated in a thickness of 400 µm at a polyurethane film laminated to a release paper. The solution of vaccine prepared as described in example 1 is sprayed evenly to the adhesive's open surface in circular areas of 10 mm and in an amount equalling 0.100 mg/ cm².

After drying a release liner is laminated to the open surface and the final patches are die-cut from the laminate in sizes of 30 mm having the vaccine area in the center. Eventually the patches are packed and sterilized with gamma radiation at 30 kGy.

### Example 6

### Cross-linked hydrogel as vehicle for vaccine

A homogeneous blend of Polyvinyl Pyrrolidone (Plasdone K 120, ISP) 15 g, polyethylene glycol 5 g and demineralised water 80 g is poured into a dish in a layer thickness of 0.5 mm. An open structured non-woven of polyester is placed on top of this layer and an additional layer of 0.5 mm of the homogeneous blend is added. Finally a layer of 30 µm thick polyurethane film is applied on top of the construct. The dish is then beta-radiated with 30 kGy for cross-linking.

After cross-linking the construct is removed from the dish and inverted. On the new top surface opposed to the polyurethane film a layer of vaccine prepared as described in example 1 is sprayed in a concentration of 0.050 mg/ cm².

The finished construct is die-cut into patches and packed in sealed packaging of metallized barrier laminate film.

### Example 7

### Cross-linked hydrogel as vehicle for vaccine

A homogenized blend of PVP K 90 (Plasdone), glycerol and water in ratios 20/15/65 is further added 1.0 part of ethoxylated (4)pentaerythritol tetraacrylate (from Sartomer) and a cross-linking photoinitiater (Darocure 1173 from CIBA) in an amount 0.5 %. To the blend is further introduced vaccine prepared as described in example 1 corresponding to 0.05 % determined by dry matter.

The blend is coated at a polyethylene film (barrier film) in a thickness of 1 mm on top of the coating a polyurethane film in a thickness of 25 µm is laminated. The coated and laminated material is in a following step cured with UV-light (Fusion UV Systems, Inc) for 60 seconds.

The laminate is die-cut to patches and packed.

### Example 8

### Lyophilised cross-linked hydrogel as vehicle for vaccine

The patch as produced in example 4 is frozen (-40 °C) and lyophilised (24 hrs) in standard laboratory equipment. The resulting porous pad is applied to the center of a hydrocolloid adhesive patch without vaccine as of example 1 leaving at least 5 mm free adhesive surrounding. Said patch will at one outer surface be covered with a polyurethane film and at the other a release liner.

The patch is packed individually in airtight pouch and sterilized by beta radiation.

### Example 9

### Lyophilised cellulose derivative as vehicle for vaccine

Vaccine (0.025 %) prepared as described in example 1 is mixed with a 5% aqueous solution of hydroxyethyl cellulose (Natrosol 250 M, pharm, Hercules Inc) and 0.5% PEG 300 and further 0.1% glycerol. The resulting blend is casted into wells of 15 mm in diameter in a layer of 0.5 mm in thickness. The tray with the wells and the casting is transferred to a freezer (-20 °C) and after frozen (24 hrs) the tray is transferred into a freeze dryer for lyophilization.

The pads of the wells are transferred to centres of die-cut adhesive patches based on a hydrocolloid adhesive as of example 8. The adhesive has an outer rim of 0.5 cm of free adhesive. Finally a release liner is applied to top of the patch.

The patch is packed in airtight packaging and sterilized by beta radiation. In use the central pad is wetted by water for improving release of the vaccine.

### Example 10

### Lyophilised cellulose derivative as vehicle for vaccine packed with water container

The patch as described in example 9 is packed in a construct having two compartments. The first compartment contains the patch with release liner but in which case the release liner does not cover the central area with the lyophilised pad containing the vaccine. The second compartment is a container for water. The two compartments are sealed together in a way that the separating cover film comprises a breakable indentation between the compartments. When the water compartment is pressed upon the indentation is broken and water will diffuse into the lyophilised pad.

The breaking of the seal will be done just before use of the patch.

### Example 11

### Hydrogel adhesive as vehicle for vaccine

A hydrogel adhesive is produced as follows. 100 g PVP K-90 is dissolved in 400 g of ethanol. The solution is further added 20 g propylene glycol and 60 g of ordinary dry potato starch and additional 40 g of water under heating. When the mixture appears homogeneous it is coated at a film backing of polyvinyl alcohol (50 µm) in a layer of 1 mm and dried. The resulting film is in the range of 300 µm.

In a second step the vaccine prepared as described in example 1 is sprayed in a concentration corresponding to 0.05 mg/ cm². The product is dried and die-cut into patches.

### Example 12

### Hydrogel adhesive as vehicle for vaccine

A hydrogel adhesive is produced according to hot melt mixing principles. 30 g PVP K 25, 4,0 g PVA (Elvanol HV from Du Pont de Nemours & Co) and 36 g glycerol is blended in a z-blade mixer at 100 °C. When the mixture appears homogeneous further 30 g de-mineralised water is added. The final blend is pressed into a thickness of 300 µm between a release paper and a polyethylene film of 50 µm.

### Example 13

### Hydrogel adhesive as vehicle for vaccine

20 g of polyvinyl-pyrrolidone (PVP K90) was mixed with 4 g of polyethyleneglycol dimethacrylat 1000 (PEG-DMA 1000) (cross-linking agent) and 1 g sodium peroxiddisulfate (NaPS) (photoinitiator) in 75 g of 0.1 M citric acid/citrate buffer pH 6.0. Propylene glycol could be added as a plastiziser (See Table of composition below).

The polymer solution was dispensed into a suitable mold in which the thickness could be varied from 0.5 - 5 mm thickness. If backing was required the polymer solution was dispensed onto the backing film covering the bottom of the mold.

The polymer solution was then cured under UV-light. The hydrogel was UV-cured under a single UV-lamp (specifications: 200 W/cm, microwave powered "D"-spectral type lamp with a conveyor speed of 0.4 m/min). A sheet hydrogel was obtained from which hydrogels of the desired diameter could be cut.

**Table 1**

| *Hydrogel composition and dimension* | | | |
|---|---|---|---|
| Type of Hydrogel | Backing | Thickness | Diameter |
| Hydrogel 1 : | | | |
| PVP K90 20g | none | | |
| PEG-DMA 4 g | | 1, 2 & 5 mm | 1, 2 & 5 cm |
| NaPS 1 g | | | |
| Citrate buffer pH 6.0 75g | PU-film* | | |
| Hydrogel 2 : | | | |
| PVP K90 20g | none | | |
| PEG-DMA 4 g | | 1, 2 & 5 mm | 1,2 & 5 cm |
| NaPS 1 g | | | |
| Propyleneglycol 10 g | PU-film* | | |
| Citrate buffer pH 6.0 65g | | | |

| | | | |
|---|---|---|---|
| * Polyurethane film 30 µm in thickness | | | |

The final adhesive is sprayed at the adhesive side with vaccine prepared as described in example 1 to a level of 0.05 mg/ cm². The material is dried, a new release liner is applied and patches are produced.

### Example 14

### Visualization of Poslntro penetration of stratum corneum with fluorescence microscopy

Human skin was obtained from a 37-year-old woman undergoing breast reduction surgery after informed consent (approved by local ethical committee). The skin was cut into 8 mm biopsies using a biopsy-punch. Each explant was placed in an 8 mm diameter punching in an agarose gel cast in a cell culture insert for 12-well format. Acridin stained PosIntro particles (10 µl, 300 µg/ml) were carefully applied as a spheric droplet on top of the epidermal layer (stratum comeum). Explants were cultured for 3 days at 37°C, 5% CO2, air-liquid interface with Dulbeccos modified Eagles medium (DMEM) and 10% FCS. The biopsies was cryofixed in OCT (TissueTek) and 4 µM sections cut in a cryomicrotome (Leica, Germany). The fluorescence signal (green) was detected in an Olympus BX60 fluorescence microscope using U-MWIB2 wide-band interfence barrier filter.

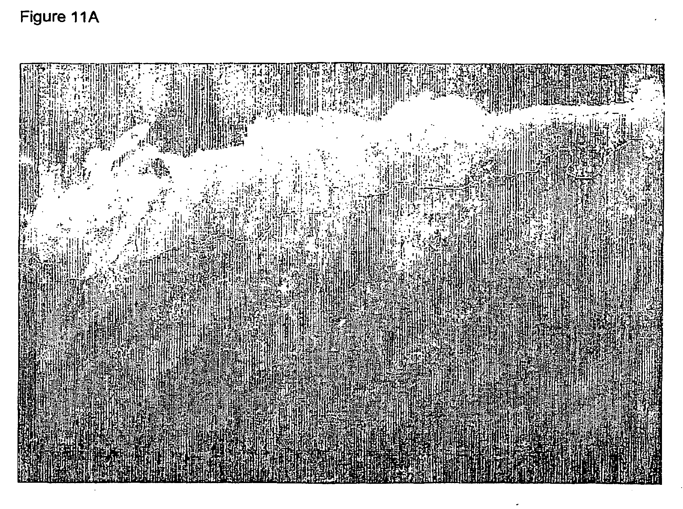

Figure 11A shows a section from the application site of Poslntro particles (100x). Basal membrane is indicated by the line.

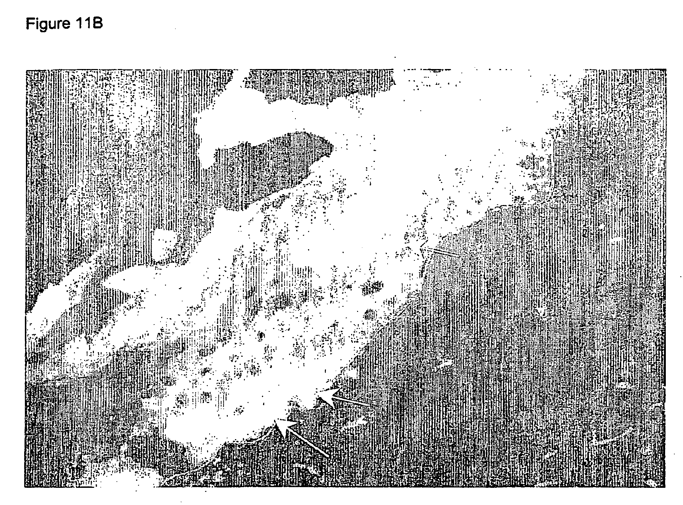

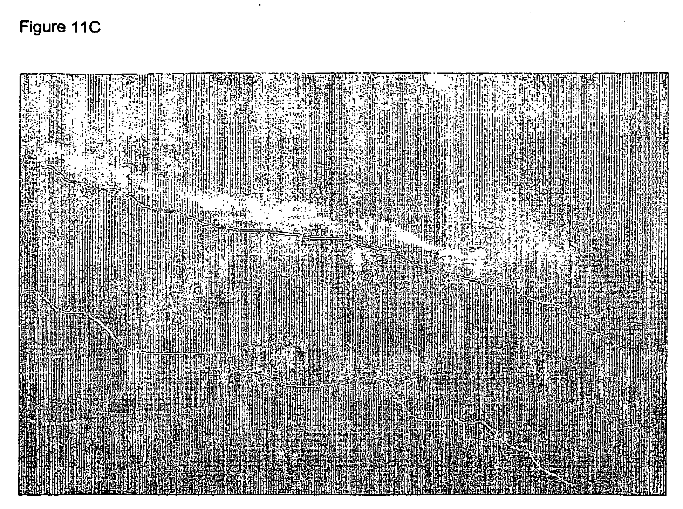

Figure 11B shows the same as Figure 11 (200x) Arrowheads indicate areas with higher fluorescence intensity, which might be attributable to Langerhans Cells.

Figure 11C shows a control section from the same slide as in Figures 11A and B, but distant from the application site of Poslntro particles. The upper line indicates beginning of stratum corneum and the lower line the basal membran

### Example 15

### Visualization of Poslntro homing to Langerhans Cells in epidermis

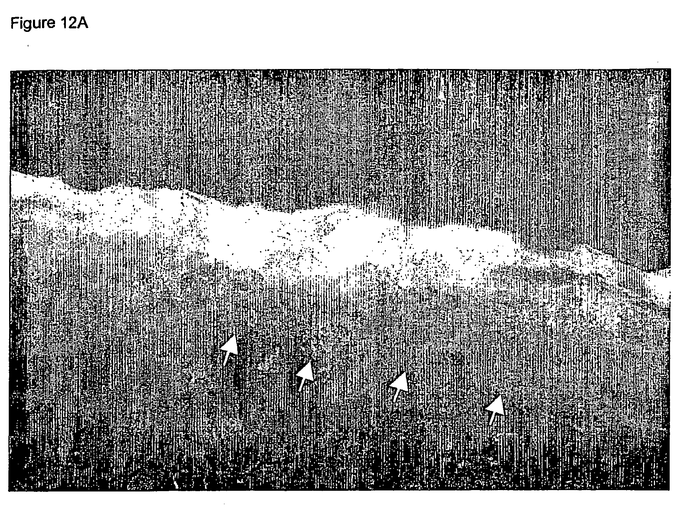

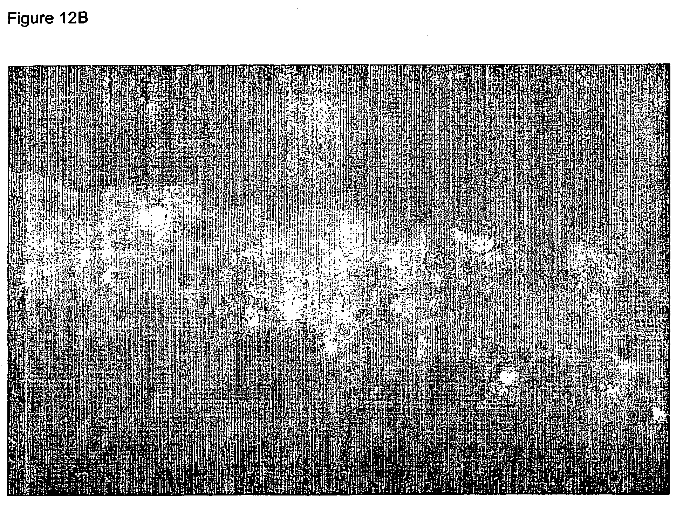

The experiment was repeated as described above and Langerhans cells were visualized by a TRITC labelled CD1a antibody (Dako, Denmark). As shown in Figure 12 the position of Langerhans cells in epidermis correlates very well with areas of Poslntro accumulation.

Figure 12A shows acridin stained Poslntro particles penetrating stratum corneum. More intense stained areas are indicated by the arrows.

Figure 12B shoes the same section as Figure 12A with TRITC-CD1a indicating the presence of Langerhans cells at the position as depicted by the arrows in Figure 12A.

### Example 16

### Visualization of Poslntro penetration through stratum corneum using confocal microscopy

Following 1 day of incubation explants with fluorescently labelled Poslntros applied on the epithelial side were cryo fixated in ice cold isopentane on dry ice in 99% ethanol. Frozen explants were divided into halves and embedded in O.C.T. compound (TissueTek, Sakura) in cryo molds.

Explants were cryo sectioned 20 µm thick and the basement membranes were labelled with primary mouse-anti-human Collagen IV a.b. (DAKO, M0785) overnight at 4°C. Next morning secundary labelling was performed with secondary Alexa Flour 488 goat-anti-mouse a.b (Molecular Probes, A-11001). Finally slides were stained 1 minute with Meyers acidic haematoxylin.

Slides were microscopically evaluated with a Zeis LSM 310 confocal microscope equipped with a 488 nm and a 543 nm laser. Basement membranes secondary labelled with Alexa Flour 488 and Acridin Orange labelled Poslntros were excitated with the 488 nm laser. Haematoxylin contrast was collected as a transmitted nonconfocal image with the 543 nm laser.

All confocal images were collected as single optical sections 8-15 µm beneath the surface of the 20-30 µm cryosections using a 40x dry objective, averaging x2, time 8 with automatic brightness and contrast and additional manual contrast enhancement. Each picture represents the merger of two confocal single optical sections and one transmitted nonconfocal image.

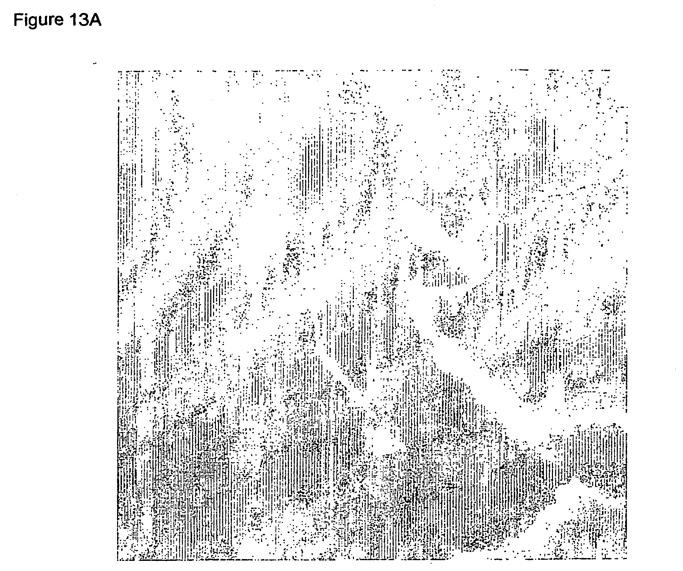

Figure 13A shows confocal microscopy of skin biopsy after incubation with acridin Poslntro for 24 hours (40x). Presence of basal membrane is indicated by a FITC labelled collagen IV antibody.

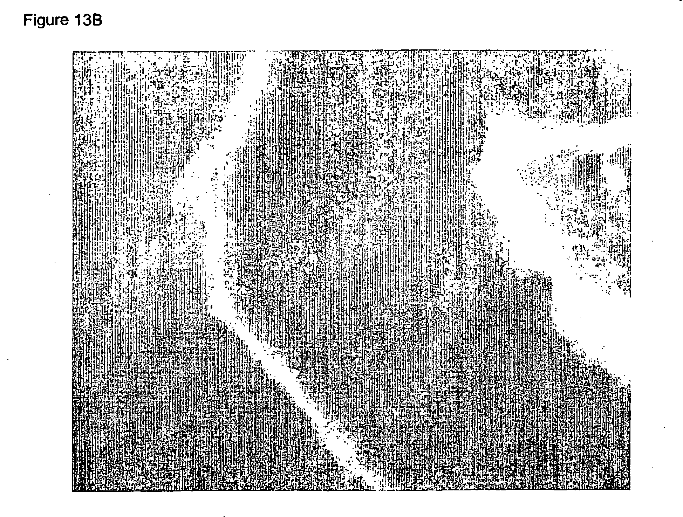

Figure 13B shows the same as Figure 13A, but in 80x, indicating penetration of stratum corneum and distribution of Poslntro particle aggregates through epidermis.

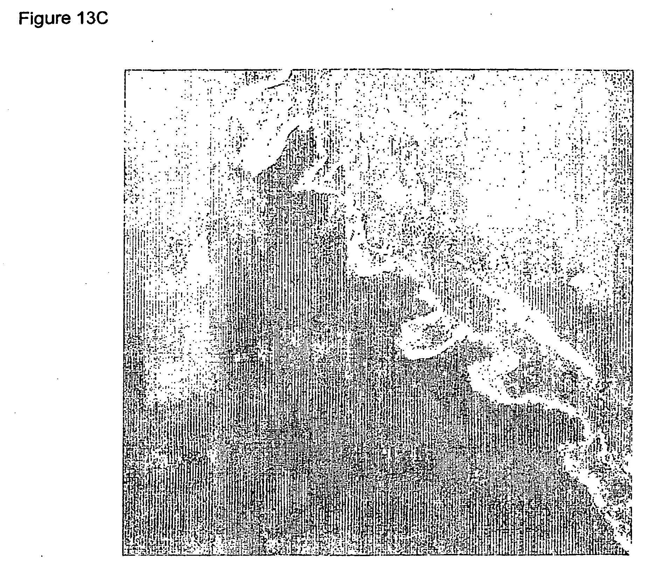

Figure 13C shows a biopsy as in Figures 13A and B without acridin Poslntro.

### Example 17

### Transfection of fibroblast cell cultures with GFP-Poslntro

L929 fibroblasts were plated on 2-well chamberslides (Nunc, Roskilde, Denmark) and grown in medium containing 90% Dulbecco's modified Eagles medium and 10% fetal calf serum. Semi-confluent cells were transfected 3 hours in serum free transfection-medium (Optimem-1, Gibco BRL, Invitrogen, UK) containing 2,5 µg/ml GFP-plasmid (gW1Z) and10 µg/ml DC-Poslntro. Control cultures were transfected with GFP-plasmid alone or GFP-plasmid and 10 µg/ml Lipofectin (Gibco, Invitrogen, UK). Transfected cells were cultured for 24 hours before evaluation by fluorescence microscopy.

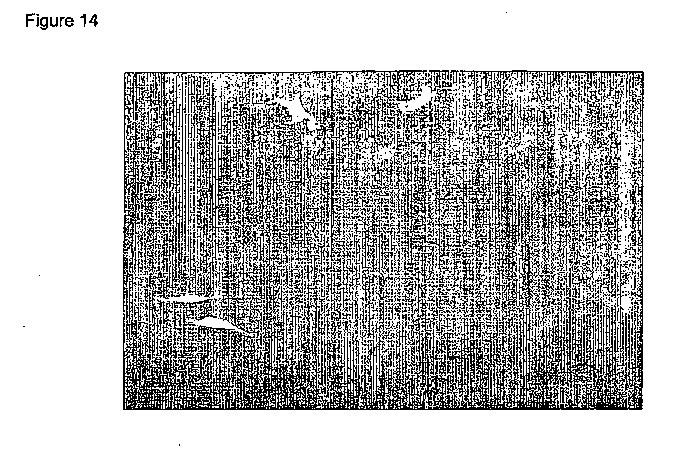

As illustrated in Figure 14, bright fluorescent GFP-expressing cultures were obtained after transfection with DC-Poslntro and GFP-plasmid. The transfection efficiency was approx 1% (compared to 10-20% for lipofectin transfected cultures) however, the appearance of cells after transfection was much healthier with DC-Poslntro compared to Lipofectin treated cultures.

### Example 18

### Transdermal Hepatits B Surface antigen vaccination.

Three rabbits received a Poslintro formulated recombinant Hepatitis B surface antigen (HBs Ag). Three weeks after immunization venues blood samples were measured for HBs Ag specific antibodies by a competition assay based on a modification of the bioMerieux, Vidas HBs antigen in vitro diagnostic system.

Recombinant HBs antigen was obtained from Aldevron, USA. Antigen was embedded intro Posintro by similar procedures as described in U.S. provisional application Serial No. 60/308,609. However, prior to embedment HBs antigen was coupled with palmetic acid in a 5:1 molar ration. Each animal received 30µg antigen divided into three hydrogel 2 patches prepared according to example 13 in a thickness of 2 mm and a diameter of 1 cm.

Twenty-four hours after immunization animals were shaved thoroughly and washed at the site of immunization. Immunization patches were placed at three different positions at the back of each animal and supported by an additional layer of Comfeel® transparent dressing, Coloplast, Denmark. As far as possible the patches were allowed to sit for 24h, however due to animal activity patches were partly released form the skin during this period.

Three weeks after immunization venous blood samples were drawn and antibody titer specific to HBs Ag measured. Antibodies were measured by mixing serial dilutions of rabbit serum with a constant amount of recombinant HBs Ag in normal human plasma (S1 standard from bioMerieux, Vidas HBs antigen kit). The mixtures were measured after 10min. incubation by bioMerieux, HBs antigen diagnostic kits in the Vidas system. Antibody titeres are given as the highest point of dilution where the detected level of HBs Ag are reduced to less than 50% compared to the addition of normal negative serum. All animals were tested negative for antibodies specific to HBs Ag prior to immunization.

| Rabbit no. | Titer prior to immunization | Test value 10⁰ | Test value 10² | Test value 10⁴ | Titer |
|---|---|---|---|---|---|
| A1 | <1 | 0.18 | 1.03 | n.d. | > 10⁰ |
| A2 | <1 | 0.03 | 0.41 | 1.04 | > 10² |
| A3 | <1 | 0.02 | 0.15 | 1.07 | > 10² |

### Table 2

Titers of Hepatitis B Surface antigen (HBs Ag). Antibody titers are given as the highest dilution of serum, 100-fold dilutions, at which the specific detection of HBs antigen is inhibited at least 50%. Test values are arbitrary units with a maximum value of 1.14.

### Example 19

### Transdermal tetanus vaccination

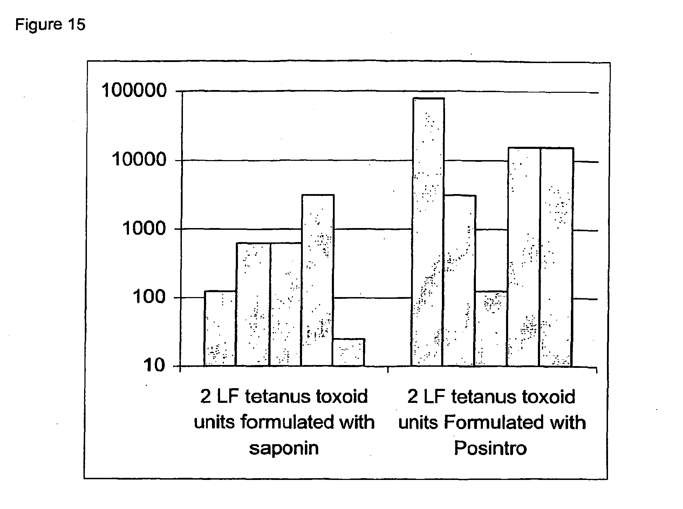

Groups of rabbits (5 animals) received a transdermal tetanus vaccine formulated with saponin or embedded into Posintro particles as illustrated in Example 2 and in a hydrogel 2 patch dimensioned as given in example 13, respectively. Blood samples were measured by ELISA 3 weeks after immunization and antibody titers determined. Titers are given in Figure 15 as the highest dilution (5-fold) at which the absorbance reading is reduced below 50% of the titer at 5⁰ dilutions.

## Claims

1. Construct for transdermal delivery of at least one immunogen to an individual comprising
a) said at least one immunogen
b) an occlusion vehicle and
c) an immunogen delivery system
wherein the immunogen delivery system is a complex comprising:
i) at least one first sterol and/or at least one second sterol,
wherein the at least one second sterol is capable of contacting a genetic determinant by means of an interaction selected from an electrostatic interaction and a hydrophobic interaction, and
wherein the at least one first sterol and/or the at least one second sterol is capable of forming a complex with at least one first saponin and/or at least one second saponin, and
ii) at least one first saponin and/or at least one second saponin,
wherein the at least one second saponin is capable of contacting a genetic determinant by means of an interaction selected from an electrostatic interaction and a hydrophobic interaction, and
wherein the at least one first saponin and/or the at least one second saponin is capable of forming a complex with at least one first sterol and/or at least one second sterol, and optionally
iii) at least one contacting group for contacting a genetic determinant by means of an interaction selected from an electrostatic interaction and a hydrophobic interaction,
with the proviso that the at least one contacting group is present when no second sterol is present in the complex and further optionally
iv) at least one lipophilic moiety.

2. Construct according to claim 1, wherein the immunogen delivery system is a complex comprising:
i) at least one first sterol and/or at least one second sterol,
wherein the at least one second sterol is capable of contacting a genetic determinant by means of an interaction selected from an electrostatic interaction and a hydrophobic interaction, and
wherein the at least one first sterol and/or the at least one second sterol is capable of forming a complex with at least one first saponin and/or at least one second saponin, and
ii) at least one first saponin and/or at least one second saponin,
wherein the at least one second saponin is capable of contacting a genetic determinant by means of an interaction selected from an electrostatic interaction and a hydrophobic interaction, and
wherein the at least one first saponin and/or the at least one second saponin is capable of forming a complex with at least one first sterol and/or at least one second sterol, and
iii) at least one contacting group for contacting a genetic determinant by means of an interaction selected from an electrostatic interaction and a hydrophobic interaction.

3. Construct according to any of the claims 1 to 2, wherein the immunogen delivery system is a complex comprising:
i) at least one first sterol and/or at least one second sterol,
wherein the at least one second sterol is capable of contacting a genetic determinant by means of an interaction selected from an electrostatic interaction and a hydrophobic interaction, and
wherein the at least one first sterol and/or the at least one second sterol is capable of forming a complex with at least one first saponin and/or at least one second saponin, and
ii) at least one first saponin and/or at least one second saponin,
wherein the at least one second saponin is capable of contacting a genetic determinant by means of an interaction selected from an electrostatic interaction and a hydrophobic interaction, and
wherein the at least one first saponin and/or the at least one second saponin is capable of forming a complex with at least one first sterol and/or at least one second sterol, and
iii) at least one contacting group for contacting a genetic determinant by means of an interaction selected from an electrostatic interaction and a hydrophobic interaction,
and further
iv) at least one lipophilic moiety.

4. Construct according to any of the claims 1 to 3, wherein the immunogen delivery system is a complex comprising:
i) at least one first sterol and/or at least one second sterol,
wherein the at least one second sterol is capable of contacting a genetic determinant by means of an interaction selected from an electrostatic interaction and a hydrophobic interaction, and
wherein the at least one first sterol and/or the at least one second sterol is capable of forming a complex with at least one first saponin and/or at least one second saponin, and
ii) at least one first saponin and/or at least one second saponin,
wherein the at least one second saponin is capable of contacting a genetic determinant by means of an interaction selected from an electrostatic interaction and a hydrophobic interaction, and
wherein the at least one first saponin and/or the at least one second saponin is capable of forming a complex with at least one first sterol and/or at least one second sterol, and optionally
iii) at least one contacting group for contacting a genetic determinant by means of an interaction selected from an electrostatic interaction and a hydrophobic interaction,
with the proviso that the at least one contacting group is present when no second sterol is present in the complex and further optionally
and further
iv) at least one lipophilic moiety.

5. Construct according to any of the claims 1 to 4, wherein the occlusion vehicle is a pressure sensitive adhesive.

6. Construct according to any of the claims 1 to 5, wherein the transdermal delivery includes delivery through a skin surface or through a mucous membrane tissue.

7. Construct according to any of the claims 1 to 6, wherein the occlusion vehicle is a absorbing pressure sensitive adhesive.

8. Construct according to any of the claims 1 to 7, wherein the occlusion vehicle is a hydrocolloid adhesive.

9. Construct according to any of the claims 1 to 7, wherein the occlusion vehicle is a hydrogel adhesive.

10. Construct according to any of the claims 1 to 7 and 9, wherein the occlusion vehicle is a cross-linked hydrogel adhesive.

11. Construct according to any of the claims 1 to 10, wherein the immunogen and the immunogen delivery system are distributed in the occlusion vehicle.

12. Construct according to claim 11, wherein the immunogen and the immunogen delivery system are distributed homogenously in the occlusion vehicle.

13. Construct according to any of the claims 1 to 11, wherein the immunogen and the immunogen delivery system are distributed on the surface of the occlusion vehicle.

14. Construct according to any of the claims 1 to 6, wherein the occlusion vehicle is a non-adherent occlusion vehicle, and further comprising a secondary adhesive, being separated from the vehicle, for skin fixation.

15. Construct according to claim 14, wherein the occlusion vehicle is dried or lyophilised and contains a carrier comprising a hydrophilic polymer substance or a grease composition.

16. Construct according to any of the claims 1 to 15, wherein the occlusion vehicle or the secondary adhesive is a covering.

17. Construct according to claim 16, wherein the occlusion vehicle or the secondary adhesive is a pad, a patch or a dressing.

18. Construct according to any of the claims 15 to 17 further comprising a reservoir of water or other appropriate solvent/diluent.

19. Construct according to claim 18, wherein the water reservoir can be broken and the water or solvent/diluent can be absorbed in the occlusion vehicle.

20. Construct according to any of the claims 1 to 19 further comprising a delivery rate controlling membrane.

21. Construct according to any of the claims 1 to 20, wherein the at least one immunogen and the immunogen delivery system are separated from each other.

22. Construct according to any of the claims 1 to 21 further comprising an enhancer for transdermal drug delivery.

23. Construct according to claim 22, wherein the enhancer is selected from alcohols, amines, phospholipids, fatty acids, surfactants, polyols, low MW-polyethylene glycol, propylene glycol, lauric acid, oleic acid, methyl laurate, ethyl oleate, N-methyl-pyrrolidone, dioctyl adipate and glycerol and low molecular weight derivatives thereof.

24. Construct according to any of the claims 1 to 23, wherein the at least one immunogen is selected in such a way that the induced immunological response is directed against one or more antigens.

25. Construct according to claim 24, wherein said one or more antigens are derived from a microorganism.

26. Construct according to claim 25, wherein said one or more antigens are derived from a pathogenic microorganism selected from a virus, a bacteria, a parasite and/or a fungus, or from a non-microbial organism selected from a vertebrate and a non-vertebrate animal.

27. Construct according to any of the claims 25 or 26, wherein the immunogen and/or antigen are derived from a virus.

28. Construct according to claim 27, wherein said one or more antigens are synthetic antigens, antigens derived from said individual or antigens derived from any species.

29. Construct according to any of the claims 27 or 28, wherein the at least one immunogen is selected in such a way that the induced immunological response confers protection in said individual against a pathogenic microorganism which said antigen or antigens are part of.

30. Construct according to any of the claims 24-25 or 29, wherein the at least one immunogen is selected in such a way that the induced immunological response may act upon subsequent exposure of the individual to said pathogenic microorganism.

31. Construct according to any of the claims 24-25 or 29-30, wherein the at least one immunogen is selected in such a way that the induced immunological response is directed against a pathogenic component produced by said pathogenic microorganism during infection of said individual.

32. Construct according to claim 31, wherein the induced immunological response is directed against bacterial toxins.

33. Construct according to claim 32, wherein the induced immunological response is directed against tetanus toxins.

34. Construct according to any of the claims 1 to 33, wherein the immunogen and/or antigen comprise
i) one or more identical or different polypeptides and/or peptides, which polypeptides and/or peptides optionally comprise posttranslational modifications,
ii) one or more identical or different lipopeptides, such as polypeptides and/or peptides chemically linked to a lipid group,
iii) one or more identical or different nucleic acid sequence or sequences, which may encode polypeptides and/or peptides, or
iv) one or more identical or different polysaccharides and/or oligosaccharides,
or combinations thereof, and wherein the immunogen may further be processed into fragments.

35. Construct according to claim 34, wherein the immunogen and/or antigen comprise
i) one or more identical or different polypeptides and/or peptides, which polypeptides and/or peptides comprise posttranslational modifications,
ii) one or more identical or different lipopeptides, such as polypeptides and/or peptides chemically linked to a lipid group,
iii) one or more identical or different nucleic acid sequence or sequences, which may encode polypeptides and/or peptides, or
iv) one or more identical or different polysaccharides and/or oligosaccharides,
or combinations thereof, and wherein the immunogen may further be processed into fragments.

36. Construct according to claim 35, wherein the immunogen and/or antigen comprises
i) one or more identical or different polypeptides and/or peptides, which polypeptides and/or peptides optionally comprise posttranslational modifications,
ii) one or more identical or different lipopeptides selected from polypeptides and/or peptides chemically linked to a lipid group,
iii) one or more identical or different nucleic acid sequence or sequences, which may encode polypeptides and/or peptides, or
iv) one or more identical or different polysaccharides and/or oligosaccharides,
or combinations thereof, and wherein the immunogen may further be processed into fragments.

37. Construct according to claim 34, wherein the immunogen and/or antigen comprises
i) one or more identical or different polypeptides and/or peptides, which polypeptides and/or peptides comprise posttranslational modifications,
ii) one or more identical or different lipopeptides selected from polypeptides and/or peptides chemically linked to a lipid group,
iii) one or more identical or different nucleic acid sequence or sequences, which may encode polypeptides and/or peptides, or
iv) one or more identical or different polysaccharides and/or oligosaccharides,
or combinations thereof, and wherein the immunogen may further be processed into fragments.

38. Construct according to any of the claims 1 to 37, wherein the immunogen and the immunogen delivery system are comprised within a vaccine formulation.

39. Process for the preparation of a construct according to any of the claims 1 to 38, comprising the steps of introducing the immunogen and the immunogen delivery system, which may or may not be comprised within a vaccine formulation, into the matrix of the occlusion vehicle or on its surface by dispersion or soaking in a solution of the vehicle or by applying to its surface, and optionally sterilising and/or drying and/or seal packaging the construct.

40. Process according to claim 39 further comprising the step of drying or lyophilisation of the immunogen and the immunogen delivery system before introducing into the vehicle.

41. Process according to any of the claims 39 or 40 further comprising the step of adding one or more enhancers for transdermal drug delivery and/or one or more plasticizers.

42. Construct according to any of the claims 1-41, having one or more compartments.

43. Construct according to claim 42 having at least two compartments,
wherein a first compartment comprises a lyophilised pad comprising the immunogen and the immunogen delivery system and a second compartment comprises water or other appropriate solvent/diluent.

44. Construct according to any of the claims 1 to 38 or 42 to 43 comprising at least two separate components.

45. Use of a construct according to any one of the claims 1 to 38 in the manufacture of a transdermal medicament for generating an immunological response in an individual.

46. Use of a construct according to any one of the claims 1 to 38 in the manufacture of a transdermal medicament for treating or preventing a condition of illness in an individual, e.g. a disease caused by infection of said individual by a pathogenic microorganism.

47. Use of a construct according to any one of the claims 1 to 38 in the manufacture of a transdermal medicament for vaccination of an individual.

## Patentansprüche

1. Konstrukt zur transdermalen Abgabe mindestens eines Immunogens auf ein Individuum, umfassend
a) das mindestens eine Immunogen
b) ein Okklusionsmedium und
c) ein Immunogen-Abgabesystem
wobei das Immunogen-Abgabesystem einen Komplex umfassend:
i) mindestens ein erstes Sterol und/oder mindestens ein zweites Sterol,
wobei das mindestens eine zweite Sterol dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Sterol und/oder das mindestens zweite Sterol dazu im Stande ist, einen Komplex mit mindestens einem ersten Saponin und/oder mindestens einem zweiten Saponin zu bilden, und
ii) mindestens ein erstes Saponin und/oder mindestens ein zweites Saponin,
wobei das mindestens eine zweite Saponin dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Saponin und/oder das mindestens zweite Saponin dazu im Stande ist, einen Komplex mit mindestens einem ersten Sterol und/oder mindestens einem zweiten Sterol zu bilden, und wahlweise
iii) mindestens eine Kontaktgruppe für das Kontaktieren einer genetischen Determinante durch eine Interaktion, die aus einer elektrostatischen Interaktion und einer hydrophoben Interaktion ausgewählt ist,
mit der Maßgabe, dass die mindestens eine Kontaktgruppe vorliegt, wenn kein zweites Sterol im Komplex vorliegt, und ferner wahlweise
iv) mindestens einen lipophilen Rest
darstellt.

2. Konstrukt nach Anspruch 1, wobei das Immunogen-Abgabesystem einen Komplex umfassend:
i) mindestens ein erstes Sterol und/oder mindestens ein zweites Sterol,
wobei das mindestens eine zweite Sterol dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Sterol und/oder das mindestens zweite Sterol dazu im Stande ist, einen Komplex mit mindestens einem ersten Saponin und/oder mindestens einem zweiten Saponin zu bilden, und
ii) mindestens ein erstes Saponin und/oder mindestens ein zweites Saponin,
wobei das mindestens eine zweite Saponin dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Saponin und/oder das mindestens zweite Saponin dazu im Stande ist, einen Komplex mit mindestens einem ersten Sterol und/oder mindestens einem zweiten Sterol zu bilden, und
iii) mindestens eine Kontaktgruppe für das Kontaktieren einer genetischen Determinante durch eine Interaktion, die aus einer elektrostatischen Interaktion und einer hydrophoben Interaktion ausgewählt ist,
darstellt.

3. Konstrukt nach einem der Ansprüche 1 bis 2, wobei das Immunogen-Abgabesystem einen Komplex umfassend:
i) mindestens ein erstes Sterol und/oder mindestens ein zweites Sterol,
wobei das mindestens eine zweite Sterol dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Sterol und/oder das mindestens zweite Sterol dazu im Stande ist, einen Komplex mit mindestens einem ersten Saponin und/oder mindestens einem zweiten Saponin zu bilden, und
ii) mindestens ein erstes Saponin und/oder mindestens ein zweites Saponin,
wobei das mindestens eine zweite Saponin dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Saponin und/oder das mindestens zweite Saponin dazu im Stande ist, einen Komplex mit mindestens einem ersten Sterol und/oder mindestens einem zweiten Sterol zu bilden, und
iii) mindestens eine Kontaktgruppe für das Kontaktieren einer genetischen Determinante durch eine Interaktion, die aus einer elektrostatischen Interaktion und einer hydrophoben Interaktion ausgewählt ist,
und ferner
iv) mindestens einen lipophilen Rest
darstellt.

4. Konstrukt nach einem der Ansprüche 1 bis 3, wobei das Immunogen-Abgabesystem einen Komplex umfassend:
i) mindestens ein erstes Sterol und/oder mindestens ein zweites Sterol,
wobei das mindestens eine zweite Sterol dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Sterol und/oder das mindestens zweite Sterol dazu im Stande ist, einen Komplex mit mindestens einem ersten Saponin und/oder mindestens einem zweiten Saponin zu bilden, und
ii) mindestens ein erstes Saponin und/oder mindestens ein zweites Saponin,
wobei das mindestens eine zweite Saponin dazu im Stande ist, durch eine Interaktion, die aus einer elektrostatischen Interaktion und einer hydrophoben Interaktion ausgewählt ist, mit einer genetischen Determinante in Kontakt zu treten, und
wobei das mindestens eine erste Saponin und/oder das mindestens zweite Saponin dazu im Stande ist, einen Komplex mit mindestens einem ersten Sterol und/oder mindestens einem zweiten Sterol zu bilden, und wahlweise
iii) mindestens eine Kontaktgruppe für das Kontaktieren einer genetischen Determinante durch eine Interaktion, die aus einer elektrostatischen Interaktion und einer hydrophoben Interaktion ausgewählt ist,
mit der Maßgabe, dass die mindestens eine Kontaktgruppe vorliegt, wenn kein zweites Sterol im Komplex vorliegt, und ferner wahlweise
und ferner
iv) mindestens einen lipophilen Rest
darstellt.

5. Konstrukt nach einem der Ansprüche 1 bis 4, wobei das Okklusionsmedium einen drucksensitiven Kleber darstellt.

6. Konstrukt nach einem der Ansprüche 1 bis 5, wobei die transdermale Abgabe die Abgabe durch eine Hautoberfläche hindurch oder durch ein Schleimhautgewebe hindurch umfasst.

7. Konstrukt nach einem der Ansprüche 1 bis 6, wobei das Okklusionsmedium ein absorbierender drucksensitiver Kleber ist.

8. Konstrukt nach einem der Ansprüche 1 bis 7, wobei das Okklusionsmedium ein Hydrokolloidkleber ist.

9. Konstrukt nach einem der Ansprüche 1 bis 7, wobei das Okklusionsmedium ein Hydrogelkleber ist.

10. Konstrukt nach einem der Ansprüche 1 bis 7 und 9, wobei das Okklusionsmedium ein vernetzter Hydrogelkleber ist.

11. Konstrukt nach einem der Ansprüche 1 bis 10, wobei das Immunogen und das Immunogen-Abgabesystem im Okklusionsmedium verteilt sind.

12. Konstrukt nach Anspruch 11, wobei das Immunogen und das Immunogen-Abgabesystem gleichmäßig im Okklusionsmedium verteilt sind.

13. Konstrukt nach einem der Ansprüche 1 bis 11, wobei das Immunogen und das Immunogen-Abgabesystem auf die Oberfläche des Okklusionsmediums verteilt sind.

14. Konstrukt nach einem der Ansprüche 1 bis 6, wobei das Okklusionsmedium ein nichthaftendes Okklusionsmedium ist, und ferner umfassend einen vom Medium getrennten, sekundären Kleber zur Fixierung an der Haut.

15. Konstrukt nach Anspruch 14, wobei das Okklusionsmedium getrocknet oder lyophilisiert ist und einen Träger umfassend eine hydrophile polymere Substanz oder eine Fettzusammensetzung enthält.

16. Konstrukt nach einem der Ansprüche 1 bis 15, wobei das Okklusionsmedium oder der sekundäre Kleber eine Abdeckung ist.

17. Konstrukt nach Anspruch 16, wobei das Okklusionsmedium oder der sekundäre Kleber eine Pelotte, ein Pflaster oder ein Verband ist.

18. Konstrukt nach einem der Ansprüche 15 bis 17, welches ferner einen Speicher von Wasser oder einem anderen geeigneten Lösungs- oder Verdünnungsmittel umfasst.

19. Konstrukt nach Anspruch 18, wobei der Wasserspeicher durchbrochen und das Wasser oder Lösungs-/Verdünnungsmittel im Okklusionsmedium absorbiert werden kann.

20. Konstrukt nach einem der Ansprüche 1 bis 19, welches ferner eine Membran zur Steuerung der Abgaberate umfasst.

21. Konstrukt nach einem der Ansprüche 1 bis 20, wobei das mindestens eine Immunogen und das Immunogen-Abgabesystem voneinander getrennt sind.

22. Konstrukt nach einem der Ansprüche 1 bis 21, welches ferner einen Verstärker zur transdermalen Abgabe eines Arzneimittels umfasst.

23. Konstrukt nach Anspruch 22, wobei der Verstärker aus Alkoholen, Aminen, Phospholipiden, Fettsäuren, Tensiden, Polyolen, Polyethylenglycol mit niedrigem Molekulargewicht, Propoylenglycol, Laurinsäure, Oleinsäure, Methyllaurat, Ethyloleat, N-Methyl-pyrrolidon, Dioctyladipat und Glycerol und deren Derivaten mit einem niedrigen Molekulargewicht ausgewählt ist.

24. Konstrukt nach einem der Ansprüche 1 bis 23, wobei das mindestens eine Immunogen derart ausgewählt ist, dass die induzierte Immunreaktion gegen ein oder mehrere Antigen(e) gerichtet ist.

25. Konstrukt nach Anspruch 24, wobei das eine oder die mehreren Antigen(e) von einem Mikroorganismus abgeleitet ist/sind.

26. Konstrukt nach Anspruch 25, wobei das eine oder die mehreren Antigen(e) von einem aus einem Virus, einem Bakterium, einem Parasiten und/oder einem Pilz ausgewählten, pathogenen Mikroorganismus oder von einem aus einem vertebraten und einem nicht vertebraten Tier ausgewählten, nicht mikrobiologischen Organismus abgeleitet ist/sind.

27. Konstrukt nach einem der Ansprüche 25 oder 26, wobei das Immunogen und/oder Antigen von einem Virus abgeleitet ist.

28. Konstrukt nach Anspruch 27, wobei das eine oder die mehreren Antigen(e) synthetische Antigene, vom Individuum abgeleitete Antigene oder von irgendeiner Art abgeleitete Antigene sind.

29. Konstrukt nach einem der Ansprüche 27 oder 28, wobei das mindestens eine Immunogen derart ausgewählt ist, dass die induzierte Immunreaktion einen Schutz gegen einen pathogenen Mikroorganismus im Individuum gewährt, von welchem Mikroorganismus das oder die Antigen(e) einen Bestandteil bilden.

30. Konstrukt nach einem der Ansprüche 24 bis 25 oder 29, wobei das mindestens eine Immunogen derart ausgewählt ist, dass die induzierte Immunreaktion bei einer nachträglichen Einwirkung des pathogenen Mikroorganismus auf das Individuum tätig wird.

31. Konstrukt nach einem der Ansprüche 25 bis 25 oder 29 bis 30, wobei das mindestens eine Immunogen derart ausgewählt ist, dass die induzierte Immunreaktion während einer Infektion des Individuums gegen einen durch den pathogenen Mikroorganismus erzeugten, pathogenen Bestandteil gerichtet ist.

32. Konstrukt nach Anspruch 31, wobei die induzierte Immunreaktion gegen bakterielle Toxine gerichtet ist.

33. Konstrukt nach Anspruch 32, wobei die induzierte Immunreaktion gegen Tetanustoxine gerichtet ist.

34. Konstrukt nach einem der Ansprüche 1 bis 33, wobei das Immunogen und/oder das Antigen
i) ein oder mehrere identische(s) oder unterschiedliche(s) Polypeptid(e) und/oder Peptid(e), welche Polypeptide und/oder Peptide wahlweise posttranslationale Modifikationen umfassen,
ii) ein oder mehrere identische(s) oder unterschiedliche(s) Lipopeptid(e), wie beispielsweise an eine Lipidgruppe chemisch gebundene Polypeptide und/oder Peptide,
iii) eine oder mehrere identische oder unterschiedliche Nukleinsäuresequenz(en), welche für Polypeptide und/oder Peptide kodieren können, oder
iv) ein oder mehrere identische(s) oder unterschiedliche(s) Polysaccharid(e) und/oder Oligosaccharid(e),
oder deren Kombinationen umfassen, und wobei ferner das Immunogen zu Fragmenten verarbeitet werden kann.

35. Konstrukt nach Anspruch 34, wobei das Immunogen und/oder Antigen
i) ein oder mehrere identische(s) oder unterschiedliche(s) Polypeptid(e) und/oder Peptid(e), welche Polypeptide und/oder Peptide posttranslationale Modifikationen umfassen,
ii) ein oder mehrere identische(s) oder unterschiedliche(s) Lipopeptid(e), wie beispielsweise an eine Lipidgruppe chemisch gebundene Polypeptide und/oder Peptide,
iii) eine oder mehrere identische oder unterschiedliche Nukleinsäuresequenz(en), welche für Polypeptide und/oder Peptide kodieren können, oder
iv) ein oder mehrere identische(s) oder unterschiedliche(s) Polysaccharid(e) und/oder Oligosaccharid(e),
oder deren Kombinationen umfassen, und wobei ferner das Immunogen zu Fragmenten verarbeitet werden kann.

36. Konstrukt nach Anspruch 35, wobei das Immunogen und/oder Antigen
i) ein oder mehrere identische(s) oder unterschiedliche(s) Polypeptid(e) und/oder Peptid(e), welche Polypeptide und/oder Peptide wahlweise posttranslationale Modifikationen umfassen,
ii) ein oder mehrere identische(s) oder unterschiedliche(s) Lipopeptid(e), welche aus an eine Lipidgruppe chemisch gebundenen Polypeptiden und/oder Peptiden ausgewählt ist/sind,
iii) eine oder mehrere identische oder unterschiedliche Nukleinsäuresequenz(en), welche für Polypeptide und/oder Peptide kodieren können, oder
iv) ein oder mehrere identische(s) oder unterschiedliche(s) Polysaccharid(e) und/oder Oligosaccharid(e),
oder deren Kombinationen umfassen, und wobei ferner das Immunogen zu Fragmenten verarbeitet werden kann.

37. Konstrukt nach Anspruch 34, wobei das Immunogen und/oder Antigen
i) ein oder mehrere identische(s) oder unterschiedliche(s) Polypeptid(e) und/oder Peptid(e), welche Polypeptide und/oder Peptide posttranslationale Modifikationen umfassen,
ii) ein oder mehrere identische(s) oder unterschiedliche(s) Lipopeptid(e), welche aus an eine Lipidgruppe chemisch gebundenen Polypeptiden und/oder Peptiden ausgewählt ist/sind,
iii) eine oder mehrere identische oder unterschiedliche Nukleinsäuresequenz(en), welche für Polypeptide und/oder Peptide kodieren können, oder
iv) ein oder mehrere identische(s) oder unterschiedliche(s) Polysaccharid(e) und/oder Oligosaccharid(e),
oder deren Kombinationen umfassen, und wobei ferner das Immunogen zu Fragmenten verarbeitet werden kann.

38. Konstrukt nach einem der Ansprüche 1 bis 37, wobei das Immunogen und das Immunogen-Abgabesystem in einer Impfstoffformulierung enthalten sind.

39. Verfahren zur Herstellung eines Konstrukts nach einem der Ansprüche 1 bis 38, umfassend die folgende Schritte: Einführen des Immunogens und des Immunogen-Abgabesystems, welche in einer Impfstoffformulierung enthalten oder nicht enthalten sein können, in eine Matrix des Okklusionsmediums oder auf dessen Oberfläche durch Dispergieren oder Tauchen in einer Lösung des Mediums oder durch Auftragen auf dessen Oberfläche, und wahlweise Sterilisation und/oder Trocknung und/oder Versiegeln des Konstruktes.

40. Verfahren nach Anspruch 39, ferner umfassend den Schritt der Trocknung oder Lyophilisation des Immunogens und des Immunogen-Abgabesystems vor dem Einführen in das Medium.

41. Verfahren nach einem der Ansprüche 39 oder 40, ferner umfassend den Schritt der Zugabe eines oder mehrerer Verstärker(s) zur transdermalen Abgabe eines Arzneimittels und/oder eines oder mehrerer Weichmacher(s).

42. Konstrukt nach einem der Ansprüche 1 bis 41, welches ein oder mehrere Kompartmente aufweist.

43. Konstrukt nach Anspruch 42, welches mindestens zwei Kompartmente aufweist, wobei ein erstes Kompartment eine das Immunogen und das Immunogen-Abgabesystem aufweisende, lyophilisierte Pelotte umfasst, und ein zweites Kompartment Wasser oder ein anderes geeignetes Lösungs- oder Verdünnungsmittel umfasst.

44. Konstrukt nach einem der Ansprüche 1 bis 38 oder 42 bis 43, welches mindestens zwei getrennte Bestandteile umfasst.

45. Anwendung eines Konstrukts nach einem der Ansprüche 1 bis 38 bei der Herstellung eines transdermalen Arzneimittels zum Erzeugen einer Immunreaktion in einem Individuum.

46. Anwendung eines Konstrukts nach einem der Ansprüche 1 bis 38 bei der Herstellung eines transdermalen Arzneimittels zur Behandlung oder Verhinderung eines Erkrankungszustandes eines Individuums, beispielsweise einer durch eine Infektion des Individuums durch einen pathogenen Mikroorganismus verursachten Erkrankung.

47. Anwendung eines Konstrukts nach einem der Ansprüche 1 bis 38 bei der Herstellung eines transdermalen Arzneimittels zur Impfung eines Individuums.

## Revendications

1. Gène hybride pour l'administration transdermique d'au moins un immunogène à un individu comprenant
a) ledit au moins un immunogène
b) un véhicule d'occlusion et
c) un système d'administration de l'immunogène
dans lequel le système d'administration de l'immunogène est un complexe comprenant :
i) au moins un premier stérol et/ou au moins un second stérol,
dans lequel le au moins un second stérol peut entrer en contact avec un déterminant génétique au moyen d'une interaction choisie parmi une interaction électrostatique et une interaction hydrophobe, et
dans lequel le au moins un premier stérol et/ou le au moins un second stérol peuvent former un complexe avec au moins une première saponine et/ou au moins une seconde saponine, et
ii) au moins une première saponine et/ou au moins une seconde saponine,
dans lequel la au moins une seconde saponine peut entrer en contact avec un déterminant génétique au moyen d'une interaction choisie parmi une interaction électrostatique et une interaction hydrophobe, et
dans lequel la au moins une première saponine et/ou la au moins une seconde saponine peuvent former un complexe avec au moins un premier stérol et/ou au moins un second stérol, et éventuellement
iii) au moins un groupe de contact pour la mise en contact avec un déterminant génétique au moyen d'une interaction choisie parmi une interaction électrostatique et une interaction hydrophobe, et
à condition que le au moins un groupe de contact soit présent lorsqu'aucun second stérol n'est présent dans le complexe et en outre éventuellement
iv) au moins un fragment lipophile.

2. Gène hybride selon la revendication 1, dans lequel le système d'administration de l'immunogène est un complexe comprenant :
i) au moins un premier stérol et/ou au moins un second stérol,
dans lequel le au moins un second stérol peut entrer en contact avec un déterminant génétique au moyen d'une interaction choisie parmi une interaction électrostatique et une interaction hydrophobe, et
dans lequel le au moins un premier stérol et/ou le au moins un second stérol peuvent former un complexe avec au moins une première saponine et/ou au moins une seconde saponine, et
ii) au moins une première saponine et/ou au moins une seconde saponine,
dans lequel la au moins une seconde saponine peut entrer en contact avec un déterminant génétique au moyen d'une interaction choisie parmi une interaction électrostatique et une interaction hydrophobe, et
dans lequel la au moins une première saponine et/ou la au moins une seconde saponine peuvent former un complexe avec au moins un premier stérol et/ou au moins un second stérol, et
iii) au moins un groupe de contact pour la mise en contact avec un déterminant génétique au moyen d'une interaction choisie parmi une interaction électrostatique et une interaction hydrophobe.

3. Gène hybride selon l'une quelconque des revendications 1 et 2, dans lequel le système d'administration de l'immunogène est un complexe comprenant :
i) au moins un premier stérol et/ou au moins un second stérol,
dans lequel le au moins un second stérol peut entrer en contact avec un déterminant génétique au moyen d'une interaction choisie parmi une interaction électrostatique et une interaction hydrophobe, et
dans lequel le au moins un premier stérol et/ou le au moins un second stérol peuvent former un complexe avec au moins une première saponine et/ou au moins une seconde saponine, et
ii) au moins une première saponine et/ou au moins une seconde saponine,
dans lequel la au moins une seconde saponine peut entrer en contact avec un déterminant génétique au moyen d'une interaction choisie parmi une interaction électrostatique et une interaction hydrophobe, et
dans lequel la au moins une première saponine et/ou la au moins une seconde saponine peuvent former un complexe avec au moins un premier stérol et/ou au moins un second stérol, et
iii) au moins un groupe de contact pour la mise en contact avec un déterminant génétique au moyen d'une interaction choisie parmi une interaction électrostatique et une interaction hydrophobe,
et en outre
iv) au moins un fragment lipophile.

4. Gène hybride selon l'une quelconque des revendications 1 à 3, dans lequel le système d'administration de l'immunogène est un complexe comprenant :
i) au moins un premier stérol et/ou au moins un second stérol,
dans lequel le au moins un second stérol peut entrer en contact avec un déterminant génétique au moyen d'une interaction choisie parmi une interaction électrostatique et une interaction hydrophobe, et
dans lequel le au moins un premier stérol et/ou le au moins un second stérol peuvent former un complexe avec au moins une première saponine et/ou au moins une seconde saponine, et
ii) au moins une première saponine et/ou au moins une seconde saponine,
dans lequel la au moins une seconde saponine peut entrer en contact avec un déterminant génétique au moyen d'une interaction choisie parmi une interaction électrostatique et une interaction hydrophobe, et
dans lequel la au moins une première saponine et/ou la au moins une seconde saponine peuvent former un complexe avec au moins un premier stérol et/ou au moins un second stérol, et éventuellement
iii) au moins un groupe de contact pour la mise en contact avec un déterminant génétique au moyen d'une interaction choisie parmi une interaction électrostatique et une interaction hydrophobe, et
à condition que le au moins un groupe de contact soit présent lorsqu'aucun second stérol n'est présent dans le complexe et en outre éventuellement
iv) au moins un fragment lipophile.

5. Gène hybride selon l'une quelconque des revendications 1 à 4, dans lequel le véhicule d'occlusion est un adhésif sensible à la pression.

6. Gène hybride selon l'une quelconque des revendications 1 à 5, dans lequel l'administration transdermique comprend l'administration à travers la surface de la peau ou à travers un tissu muqueux.

7. Gène hybride selon l'une quelconque des revendications 1 à 6, dans lequel le véhicule d'occlusion est un adhésif absorbant sensible à la pression.

8. Gène hybride selon l'une quelconque des revendications 1 à 7, dans lequel le véhicule d'occlusion est un adhésif hydrocolloïde.

9. Gène hybride selon l'une quelconque des revendications 1 à 7, dans lequel le véhicule d'occlusion est un adhésif d'hydrogel.

10. Gène hybride selon l'une quelconque des revendications 1 à 7 et 9, dans lequel le véhicule d'occlusion est un adhésif d'hydrogel réticulé.

11. Gène hybride selon l'une quelconque des revendications 1 à 10, dans lequel l'immunogène et le système d'administration de l'immunogène sont distribués dans le véhicule d'occlusion.

12. Gène hybride selon la revendication 11, dans lequel l'immunogène et le système d'administration de l'immunogène sont distribués de manière homogène dans le véhicule d'occlusion.

13. Gène hybride selon l'une quelconque des revendications 1 à 11, dans lequel l'immunogène et le système d'administration de l'immunogène sont distribués sur la surface du véhicule d'occlusion.

14. Gène hybride selon l'une quelconque des revendications 1 à 6, dans lequel le véhicule d'occlusion est un véhicule d'occlusion non adhérent, et comprenant en outre un adhésif secondaire, étant séparé du véhicule, pour fixation à la peau.

15. Gène hybride selon la revendication 14, dans lequel le véhicule d'occlusion est séché ou lyophilisé et contient un vecteur qui comprend une substance polymère hydrophile ou une composition de graisse.

16. Gène hybride selon l'une quelconque des revendications 1 à 15, dans lequel le véhicule d'occlusion ou l'adhésif secondaire est un revêtement.

17. Gène hybride selon la revendication 16, dans lequel le véhicule d'occlusion ou l'adhésif secondaire est un tampon, un timbre ou un pansement.

18. Gène hybride selon l'une quelconque des revendications 15 à 17, comprenant en outre un réservoir d'eau ou d'un autre solvant/diluant approprié.

19. Gène hybride selon la revendication 18, dans lequel le réservoir d'eau peut être brisé et l'eau ou le solvant/diluant peut être absorbé dans le véhicule d'occlusion.

20. Gène hybride selon l'une quelconque des revendications 1 à 19, comprenant en outre une membrane de régulation de la vitesse d'administration.

21. Gène hybride selon l'une quelconque des revendications 1 à 20, dans lequel le au moins un immunogène et le système d'administration de l'immunogène sont séparés l'un de l'autre.

22. Gène hybride selon l'une quelconque des revendications 1 à 21, comprenant en outre un amplificateur pour l'administration transdermique de médicaments.

23. Gène hybride selon la revendication 22, dans lequel l'amplificateur est choisi parmi des alcools, des amines, des phospholipides, des acides gras, des agents tensioactifs, des polyols, un polyéthylèneglycol à faible PM, un propylèneglycol, l'acide laurique, l'acide oléique, le laurate de méthyle, l'oléate d'éthyle, la N-méthyl-pyrrolidone, l'adipate de dioctyle et un glycérol et des dérivés à faible poids moléculaire de ceux-ci.

24. Gène hybride selon l'une quelconque des revendications 1 à 23, dans lequel le au moins un immunogène est choisi de sorte que la réponse immunitaire induite est dirigée contre un ou plusieurs antigènes.

25. Gène hybride selon la revendication 24, dans lequel lesdits un ou plusieurs antigènes sont dérivés d'un microorganisme.

26. Gène hybride selon la revendication 25, dans lequel lesdits un ou plusieurs antigènes sont dérivés d'un microorganisme pathogène choisi parmi un virus, une bactérie, un parasite et/ou un champignon, ou d'un organisme non microbien choisi parmi un animal vertébré et un animal invertébré.

27. Gène hybride selon l'une quelconque des revendications 25 ou 26, dans lequel l'immunogène et/ou l'antigène sont dérivés d'un virus.

28. Gène hybride selon la revendication 27, dans lequel lesdits un ou plusieurs antigènes sont des antigènes synthétiques, des antigènes dérivés dudit individu ou des antigènes dérivés d'une quelconque espèce.

29. Gène hybride selon l'une quelconque des revendications 27 ou 28, dans lequel le au moins un immunogène est choisi de sorte que la réponse immunitaire induite confère une protection audit individu contre un microorganisme pathogène dont ledit ou lesdits antigènes font partie.

30. Gène hybride selon l'une quelconque des revendications 24 et 25 ou 29, dans lequel le au moins un immunogène est choisi de sorte que la réponse immunitaire induite peut agir lors d'une exposition ultérieure de l'individu audit microorganisme pathogène.

31. Gène hybride selon l'une quelconque des revendications 24 et 25 ou 29 et 30, dans lequel le au moins un immunogène est choisi de sorte que la réponse immunitaire induite est dirigée contre un composant pathogène produit par ledit microorganisme pathogène pendant une infection dudit individu.

32. Gène hybride selon la revendication 31, dans lequel la réponse immunitaire induite est dirigée contre des toxines bactériennes.

33. Gène hybride selon la revendication 32, dans lequel la réponse immunitaire induite est dirigée contre des toxines tétaniques.

34. Gène hybride selon l'une quelconque des revendications 1 à 33, dans lequel l'immunogène et/ou l'antigène comprennent
i) un ou plusieurs polypeptides et/ou peptides identiques ou différents, lesquels polypeptides et/ou peptides comprennent éventuellement des modifications posttranslationnelles,
ii) un ou plusieurs lipopeptides identiques ou différents, comme des polypeptides et/ou des peptides chimiquement liés à un groupe lipide,
iii) une ou plusieurs séquences d'acides nucléiques identiques ou différentes, qui peuvent coder des polypeptides et/ou des peptides, ou
iv) un ou plusieurs polysaccharides et/ou oligosaccharides identiques ou différents,
ou des combinaisons de ceux-ci, et dans lequel l'immunogène peut en outre être transformé en fragments.

35. Gène hybride selon la revendication 34, dans lequel l'immunogène et/ou l'antigène comprennent
i) un ou plusieurs polypeptides et/ou peptides identiques ou différents, lesquels polypeptides et/ou peptides comprennent des modifications posttranslationnelles,
ii) un ou plusieurs lipopeptides identiques ou différents, comme des polypeptides et/ou des peptides chimiquement liés à un groupe lipide,
iii) une ou plusieurs séquences d'acides nucléiques identiques ou différentes, qui peuvent coder des polypeptides et/ou des peptides, ou
iv) un ou plusieurs polysaccharides et/ou oligosaccharides identiques ou différents,
ou des combinaisons de ceux-ci, et dans lequel l'immunogène peut en outre être transformé en fragments.

36. Gène hybride selon la revendication 35, dans lequel l'immunogène et/ou l'antigène comprennent
i) un ou plusieurs polypeptides et/ou peptides identiques ou différents, lesquels polypeptides et/ou peptides comprennent éventuellement des modifications posttranslationnelles,
ii) un ou plusieurs lipopeptides identiques ou différents choisis parmi des polypeptides et/ou des peptides chimiquement liés à un groupe lipide,
iii) une ou plusieurs séquences d'acides nucléiques identiques ou différentes, qui peuvent coder des polypeptides et/ou des peptides, ou
iv) un ou plusieurs polysaccharides et/ou oligosaccharides identiques ou différents,
ou des combinaisons de ceux-ci, et dans lequel l'immunogène peut en outre être transformé en fragments.

37. Gène hybride selon la revendication 34, dans lequel l'immunogène et/ou l'antigène comprennent
i) un ou plusieurs polypeptides et/ou peptides identiques ou différents, lesquels polypeptides et/ou peptides comprennent des modifications posttranslationnelles,
ii) un ou plusieurs lipopeptides identiques ou différents choisis parmi des polypeptides et/ou des peptides chimiquement liés à un groupe lipide,
iii) une ou plusieurs séquences d'acides nucléiques identiques ou différentes, qui peuvent coder des polypeptides et/ou des peptides, ou
iv) un ou plusieurs polysaccharides et/ou oligosaccharides identiques ou différents,
ou des combinaisons de ceux-ci, et dans lequel l'immunogène peut en outre être transformé en fragments.

38. Gène hybride selon l'une quelconque des revendications 1 à 37, dans lequel l'immunogène et le système d'administration de l'immunogène sont compris dans une formulation vaccinale.

39. Procédé pour la préparation d'un gène hybride selon l'une quelconque des revendications 1 à 38, comprenant les étapes d'introduction de l'immunogène et du système d'administration de l'immunogène, qui peut ou non être compris dans une formulation vaccinale, dans la matrice du véhicule d'occlusion ou à sa surface par dispersion ou par immersion dans une solution du véhicule ou par application à sa surface, et éventuellement de stérilisation et/ou de séchage et/ou d'emballage hermétique du gène hybride.

40. Procédé selon la revendication 39, comprenant en outre l'étape de séchage ou de lyophilisation de l'immunogène et du système d'administration de l'immunogène avant de les introduire dans le véhicule.

41. Procédé selon l'une quelconque des revendications 39 ou 40, comprenant en outre l'étape d'ajout d'un ou plusieurs amplificateurs pour l'administration transdermique de médicaments et/ou d'un ou plusieurs plastifiants.

42. Gène hybride selon l'une quelconque des revendications 1 à 41, comprenant un ou plusieurs compartiments.

43. Gène hybride selon la revendication 42, comprenant au moins deux compartiments, dans lequel un premier compartiment comprend un tampon lyophilisé qui comprend l'immunogène et le système d'administration de l'immunogène et un second compartiment comprend de l'eau ou un autre solvant/diluant approprié.

44. Gène hybride selon l'une quelconque des revendications 1 à 38 ou 42 et 43, comprenant au moins deux composants séparés.

45. Utilisation d'un gène hybride selon l'une quelconque des revendications 1 à 38 dans la fabrication d'un médicament transdermique pour la génération d'une réponse immunitaire chez un individu.

46. Utilisation d'un gène hybride selon l'une quelconque des revendications 1 à 38 dans la fabrication d'un médicament transdermique pour le traitement ou la prévention d'un état pathologique chez un individu, par ex., une maladie causée par infection dudit individu par un microorganisme pathogène.

47. Utilisation d'un gène hybride selon l'une quelconque des revendications 1 à 38 dans la fabrication d'un médicament transdermique pour la vaccination d'un individu.
